# EUROPEAN PATENT APPLICATION

(11) **EP 2 246 330 A1**
(43) Date of publication of application: **03.11.2010**
(21) Application number: 09159156.0
(22) Date of filing: 30.04.2009
(51) Int. Cl.: C07D 219/06, C07D 335/12, C07D 401/12, C07D 409/12, C08K 5/45, C09D 11/10

(54) **New photoinitiators**

(71) Applicant: Siegwerk Benelux SA, 2880 Bornem (BE)
(72) Inventor: Noirot, Pierre Antoine, 74800 Arenthon (FR); Carni, Matthieu, Shanghai 200030 (CN); Marsille, Thierry, 74800 Saint Pierre en Faucigny (FR); Catherin, Gilles, 01630 Saint Genis Pouilly (FR)
(74) Representative: Hepp, Dieter

(57) **Abstract**

The present invention is related to novel photoinitiators, in particular to photoinitiators comprising amino groups within the molecule.

## Description

The present invention is related to novel compounds which are useful as photoinitiators in coating compositions, preferably printing inks.

Energy-curable compositions, such as UV-curable compositions, generally comprise a photoinitiator for starting/enhancing the polymerization reaction of the polymerizable material, which usually comprises ethylenically unsaturated moieties. Those photoinitiators must exhibit a good curing activity, low odour and good compatibility with the other components of the coating composition. In particular for food packaging applications, there are increasing legislative requirements a photoinitiator has to fulfil. Furthermore, the photoinitiators should be available at reasonable or low cost.

Accordingly, there has been research going on in the recent years for developing new photoinitiators meeting the above requirements. For example, in WO 03/033492, a series of photoinitiators is described.

It was the object of the present invention to provide novel photoinitiators meeting the above requirements.

This object has been solved according to the present invention by a series of photoinitiators as defined in the claims. In particular, the present invention is related to photoinitiators of the formula (I)

(PI-Sp)ₙ-BB (I)

wherein
- PI: is a thioxanthone or acridone moiety optionally com- prising additional substituents further to the Sp moi- ety
- Sp: is a spacer unit which, if PI is a thioxanthone moiety, is selected from the group consisting of 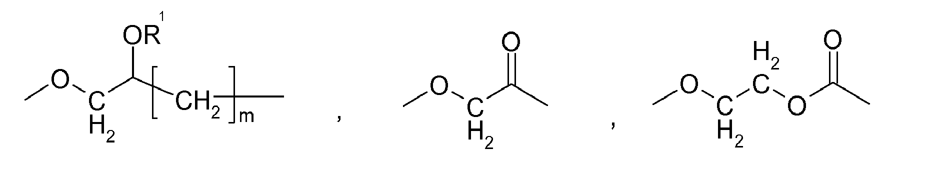 or, if PI is an acridone moiety, is 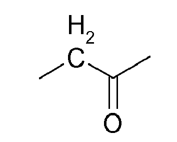 wherein
R1 is H, an optionally substituted linear or branched C₁₋₈ alkyl residue, an optionally substituted C₃₋₁₀ cycloalkyl residue or an acyl residue;
m is 0 or 1
- BB: is a backbone moiety selected from the group consisting of optionally substituted heteroaliphatic or heteroali- cyclic polyoxyethylene amines or polyoxypropylene amines, optionally substituted urethanes, optionally substituted pentaerythritol-based polyols, optionally substituted polyoxyethylene oxides and optionally sub- stituted polyoxypropylene oxides; and
- n: is 1, 2 or 3;
with the proviso that if Sp is 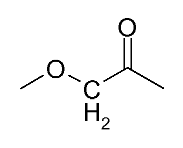 and PI is thioxanthone and does not comprise additional substituents besides Sp, then BB is not
a moiety from the group consisting of [0 (CH^{R}2CH^{R}1₎ a] y-Q, -[O(CH₂)_{b}CO]y-Q, or-[O(CH₂)_{b}CO_{(y-1)}[O(CHR²CHR¹)ₐ]-Q, where one of R¹ and R² represents a hydrogen atom and the other represents a hydrogen atom, a methyl group or an ethyl group; a is a number from 1 to 2; b is a number from 4 to 5; Q is a residue of a polyhydroxy compound having 2 to 6 hydroxy groups; y is a number from 3 to 10.

The present invention discloses new structures of photoinitiators. It has been surprisingly found that the novel compounds of the present invention are suitable photoinitiators. This could not be foreseen from the teaching of WO 03/033492 which is limited to specific structures. Thus, the present invention widely expands the range of compounds to be considered as photoinitiators in coating compositions, preferably printing inks.

According to a preferred embodiment of the present invention, it has been surprisingly found that it is also possible to insert amine groups into the backbone of the photoinitiators, and that these amine groups enhance the curing characteristics of the compounds of the present invention. Most preferably, the amine groups are tertiary amine groups. According to the present invention, the amine groups can be part of the main backbone of the compounds of the present invention, or alternatively they can be introduced into the compounds as side or terminating groups.

According to another embodiment of the present invention, novel nitrogen-free photoinitiators are also provided showing also good curing and compatibility characteristics.

Thus, the preferred backbone moieties BB of the present invention are selected from the group consisting of 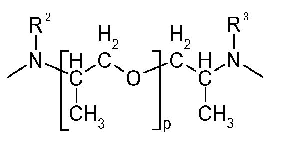 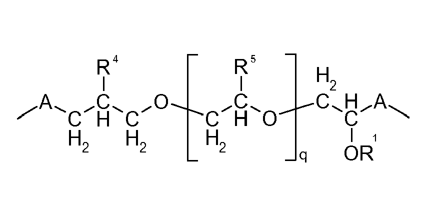 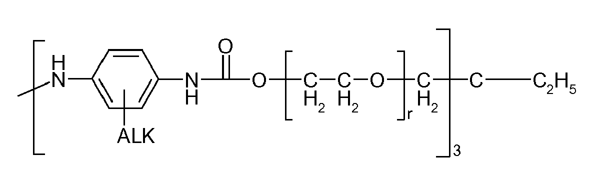 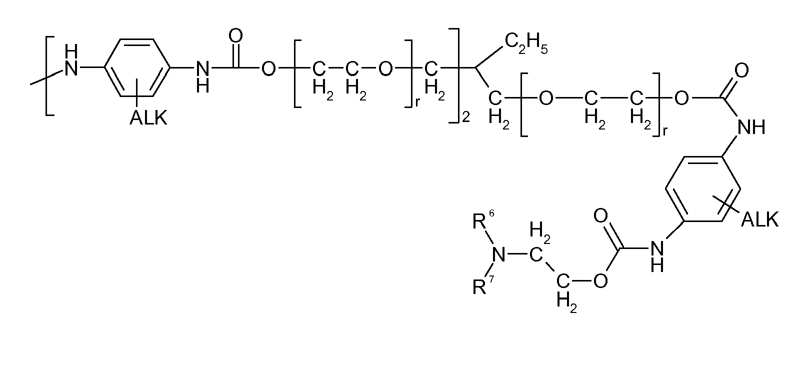 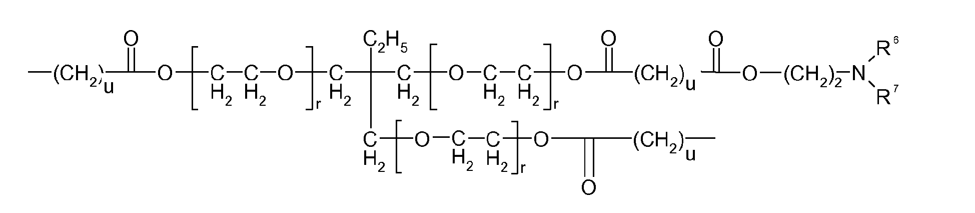 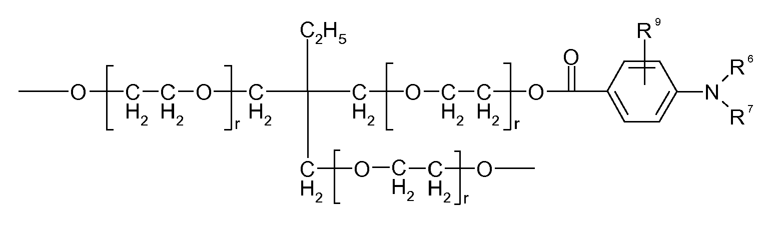 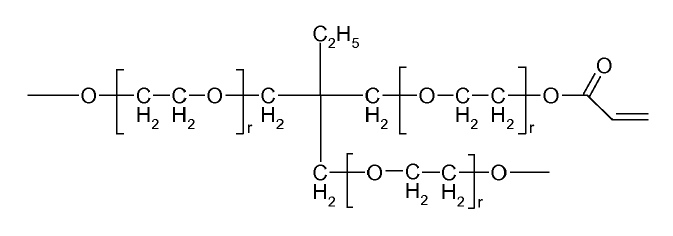 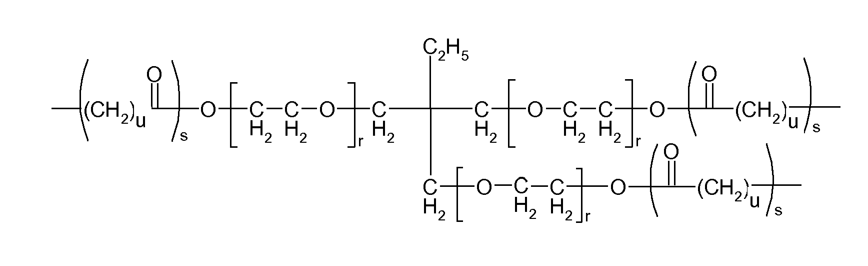 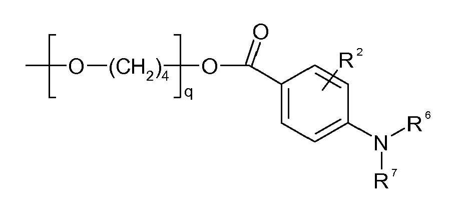 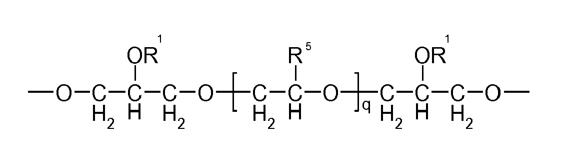 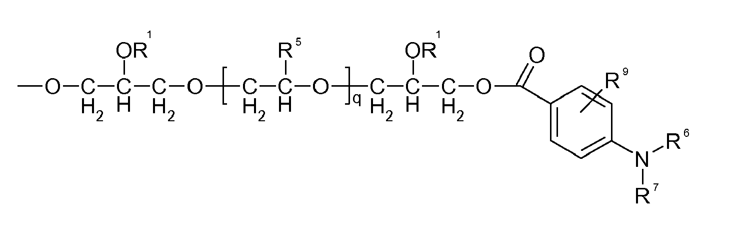 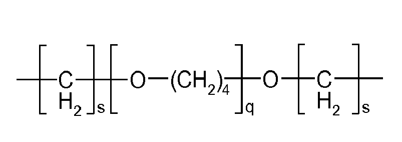 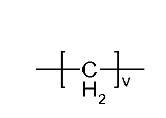 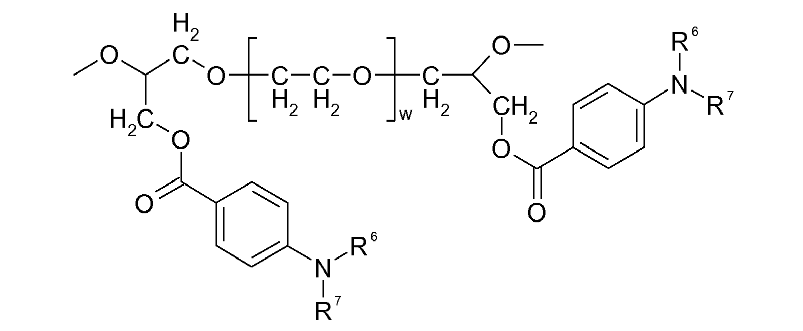 wherein
- R¹: is as defined above;
- R² and R³: are the same or different and denote H, an op- tionally substituted linear or branched C₁₋₈ alkyl residue, an optionally substituted C₃₋₁₀ cycloal- kyl residue, an acyl residue, or an optionally substituted C₁₋₈ alkylamine residue;
- R⁴: is H or Hydroxy or Alkoxy
- R⁵: is H or CH₃
- R⁶ and R⁷: are the same or different and denote H, an op- tionally substituted linear or branched C₁₋₈ alkyl residue, or an optionally substituted C₃₋₁₀ cycloalkyl residue;
- A: is NR⁸ or an optionally substituted piperazine moiety, wherein R⁸ is H, an optionally substituted linear or branched C₁₋₈ alkyl residue, an optionally substituted C₃₋₁₀ cycloalkyl residue, an acyl residue, or an optionally substituted C₁₋₈ al- kylamine residue;
- ALK: is an optionally substituted linear or branched C₁₋₈ alkyl residue;
- R⁹: is H or a linear or branched C₁₋₈ alkyl residue, preferably linear or branched C₁₋₄ alkyl residue
- p: is an integer from 1 to 10, preferably from 3 to 6;
- q: is an integer from 1 to 15, preferably from 3 to 10;
- r: is in the range from 2 to 3;
- s: is independently at each position 0 or 1;
- u: is an integer from 1 to 6;
- v: is an integer from 4 to 15;
- w: is an integer from 2 to 15, preferably from 3 to 10.

According to the present invention, thioxanthone derivatives are preferred, i.e. PI is preferably a thioxanthone.

According to the present invention, it has been found that the photoreactivity of the present compounds is best when the Sp moiety linking the thioxanthone moiety to the polymeric backbone is in 2-position at the thioxanthone ring.

In contrast to the teaching of WO 03/033492 it has been surprisingly found that the photoreactivity of the compounds of the invention can be enhanced if there is at least one additional substituent, beside the Sp moiety, at the PI moiety. According to a most preferred embodiment, it has been found that an additional substituent, in particular a C₁₋₄ alkyl moiety, preferably a methyl or isopropyl moiety, especially an isopropyl moiety, in 4 position of the thioxanthone moiety has a favourable effect.

Thus, according to a preferred embodiment of the present invention, the moiety PI is substituted with at least one residue selected from the group consisting of linear or branched C₁₋₈ alkyl, preferably linear or branched C₁₋₄ alkyl, linear or branched C₁₋₈ alkoxy, preferably linear or branched C₁₋₄ alkoxy, or halogen, preferably Cl. The linear or branched C₁₋₈ alkyl, preferably linear or branched C₁₋₄ alkyl residue is particularly preferred.

The synthesis of substituted thioxanthones or acridones is known to the skilled person. Several substituted thioxanthones or acridones are commercially available.

According to a preferred embodiment of the present invention, substituted thioxanthones are manufactured by the reaction of thiosalicylic acid (commercially available from e.g. Sigma Aldrich) with respectively substituted phenol derivatives. For example, the synthesis of 2-Hydroxy-4-isopropylthioxanthon is accomplished by the following route:

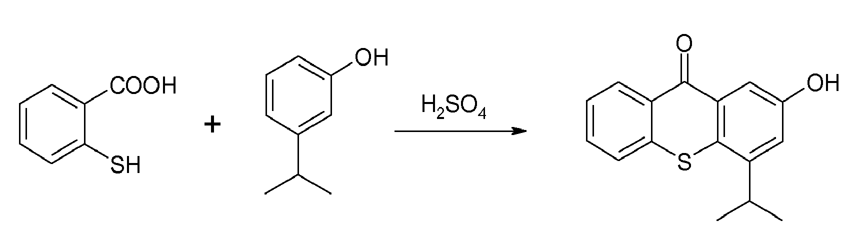

The reaction is carried out in the presence of a strong acid such as concentrated sulfuric acid under heating to about 60 to 100°C.

Likewise, acridone derivatives may be formed by reaction of an aniline derivative with a respective 2-halocarboxy benzene derivative.

The spacer units can be attached to the thioxanthone or acridone moiety via well-known reactions.

The spacer unit 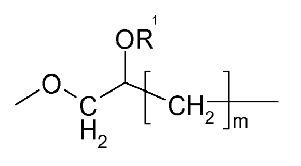 wherein m=1, may be formed by reaction of a 2-hydroxy thioxanthone derivative with epichlorohydrine and subsequent reaction with a nucleophilic group (Nu) of the backbone unit BB:

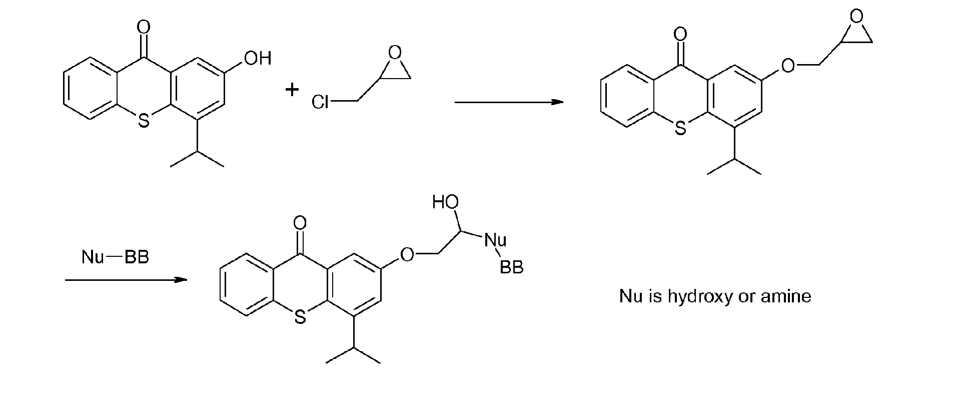

Likewise, if m=0, then the spacer unit can be generated by reacting a 2-hydroxy thioxanthone derivative with a halo acetaldehyde, e.g. chloro acetaldehyde, and subsequent reaction with a nucleophilic group (Nu) of the backbone unit BB.

The spacer unit 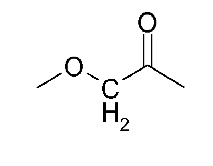 may be conveniently prepared by reaction of a 2-hydroxy thioxanthone derivative with a halo acetic acid derivative, such as bromo acetic acid ethyl ester, and subsequent reaction with a nucleophilic group (Nu) of the backbone unit BB:

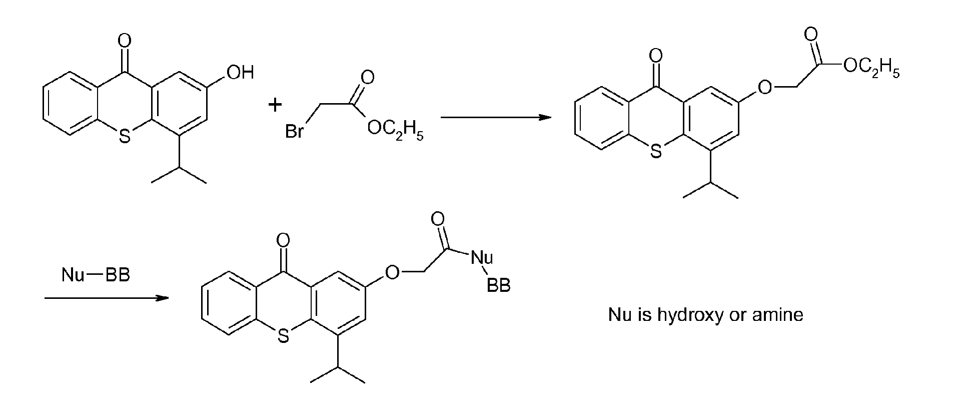

Also the reaction between an α-halocarboxylic acid derivative and a hydroxy-containing compound is well-known. It is generally carried out under heating in the presence of a base such as sodium hydroxide.

The spacer unit 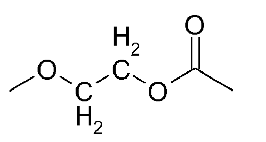 may be prepared by reacting a 2-hydroxy thioxanthone derivative with e.g. oxirane or ethylene glycol, and subsequent reaction with a carboxylic moiety (or a carboxylic derivative such as a carboxylic acid halogenide) of the backbone unit BB.

The spacer unit 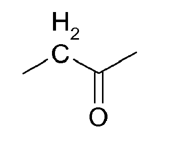 is used according to the present invention solely for acridone derivatives where the Sp unit is attached to the nitrogen atom in the heterocyclus. Said attachment can be generated by reaction a respective acridone derivative with e.g. a α-halo acetic acid derivative, such as bromo acetic acid ethyl ester, and subsequent reaction with a nucleophilic group (Nu) of the backbone unit BB:

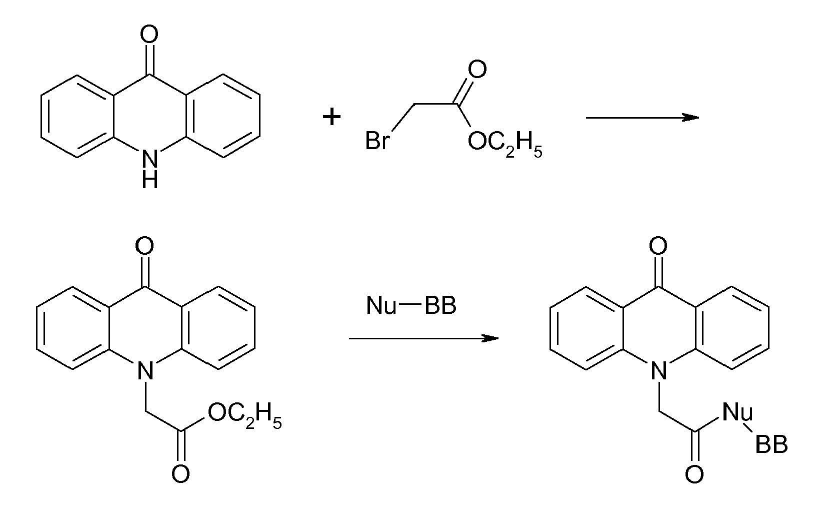

Also the reaction between an α-halocarboxylic acid derivative and an amine-containing compound is well-known. It is generally carried out under heating in the presence of a base such as sodium hydroxide.

As mentioned above, according to a particularly preferred embodiment of the present invention the backbone unit comprises at least one amine moiety. It has been surprisingly found that such compounds are not only good photoinitiators, which as such was not foreseeable, but that the insertion of such amine groups led to an enhancement of the photoreactivity of the compounds.

According to an especially preferred embodiment of the present invention, such amino group is a tertiary amino group.

A preferred group of compounds of said embodiment is characterized by the formula 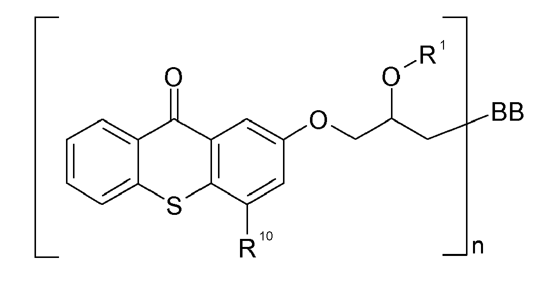 wherein
- R¹: is H, 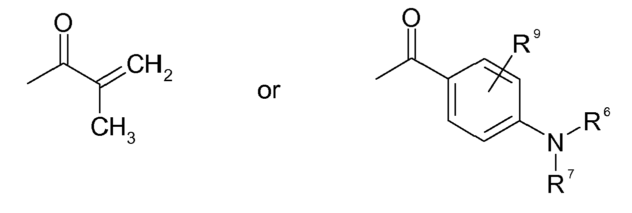 wherein
R⁶ and R⁷ are as defined above, preferably both CH₃;
R⁹ is as defined above, preferably H or CH₃.
- R¹⁰: is H or a linear or branched C₁₋₈ alkyl residue, pref- erably linear or branched C₁₋₄ alkyl residue, most pref- erably CH₃;
- BB: is selected from the group consisting of 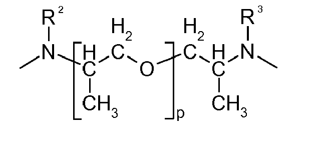 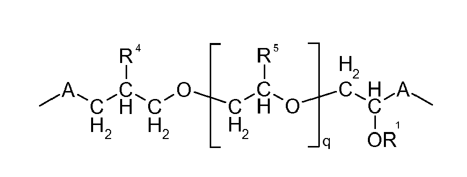 wherein
R1 is H ; 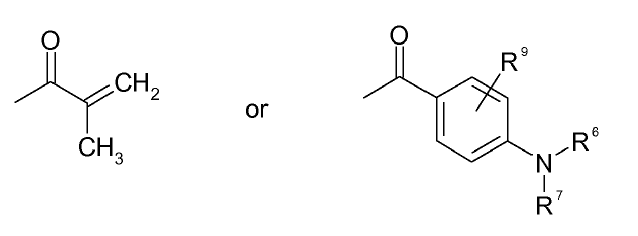 R² and R³ are the same or different and denote H, a linear or branched C₁₋₈ alkyl residue, preferably linear or branched C₁₋₄ alkyl residue, a linear or branched C₁₋₈ alkylamine residue, preferably linear or branched C₁₋₄ alkylamine residue, 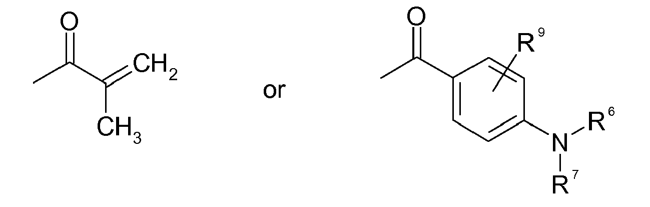 wherein R⁶, R⁷ and R⁹ are as defined above;
p is an integer from 1 to 10, preferably from 3 to 6, most preferably 3 or 5;
q is an integer from 1 to 15, preferably from 3 to 10, most preferably 9;
A is NR⁸ or an optionally substituted piperazine or piperazine methylene moiety, wherein R⁸ is H, a linear or branched C₁₋₈ alkyl residue, preferably linear or branched C₁₋₄ alkyl residue, a linear or branched C₁₋₈ alkylamine residue, preferably linear or branched C₁₋₄ alkylamine residue, 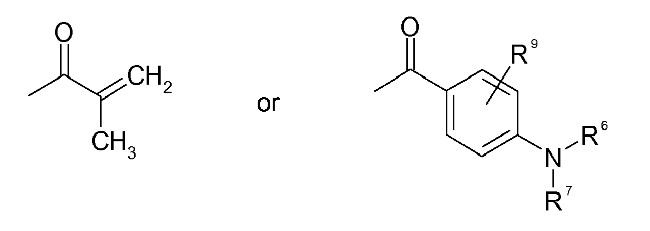 wherein R⁶, R⁷ and R⁹ are as defined above;
R⁴ is H or Hydroxy;
R⁵ is H or CH₃; and
- n: is 2.

In this group of compounds, the amine functionality may be present both in the main backbone as well as in side or terminating groups.

The PI-Sp unit to be used here is 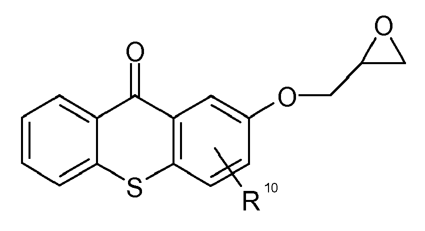 which synthesis has been described above.

The backbone units 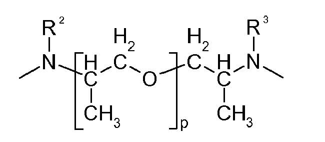 are derived from the commercially available polyoxypropylene amines such as Jeffamine D230 or Jeffamine D400.

The backbone units 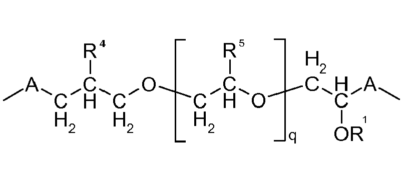 are conveniently prepared by first reacting the amine moiety A with the PI-Sp unit, followed by reaction of the free amino group with e.g. a polyoxyethyleneoxide, as exemplified for the preparation of the following compound:

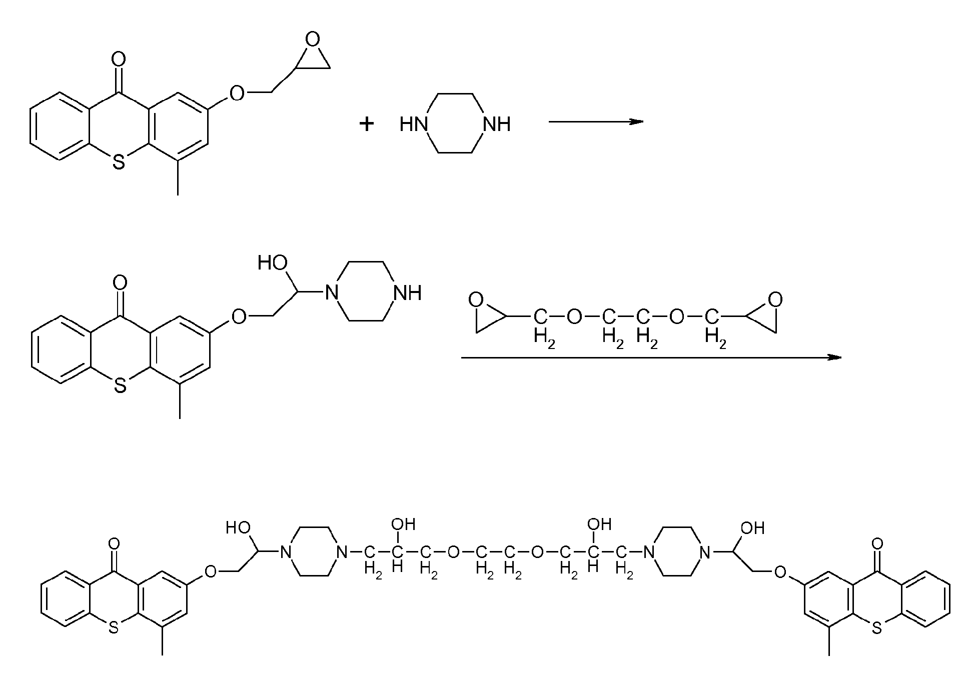

Preferred examples of compounds of this group are:

| | |
|---|---|
| i) | 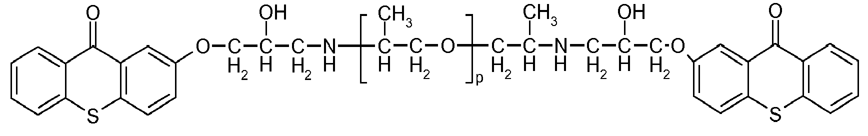 |
| ii) | 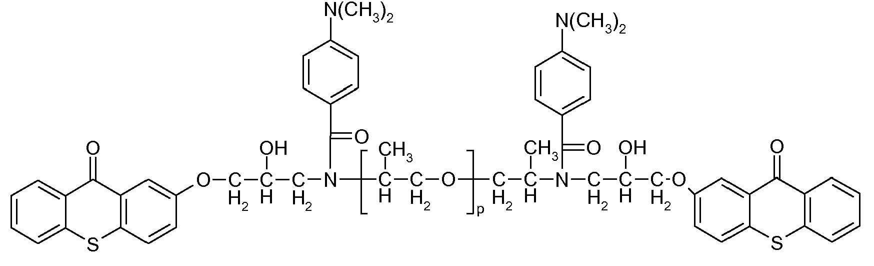 |
| iii) | 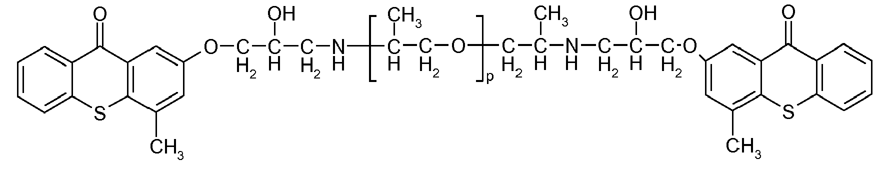 |
| iv) | 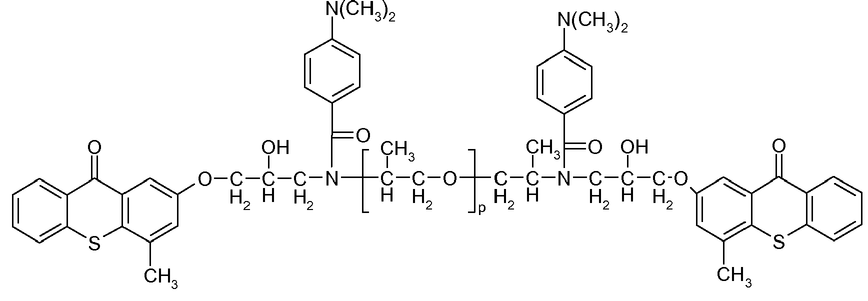 |
| v) | 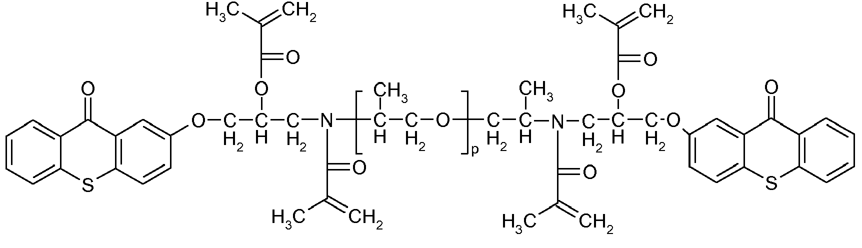 |
| vi) | 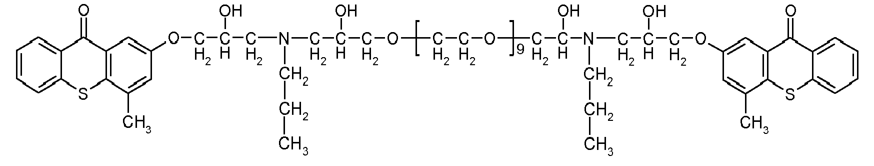 |
| vii) | 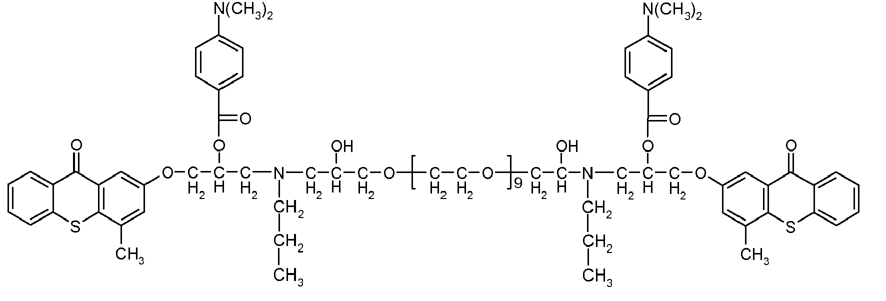 |
| viii) | 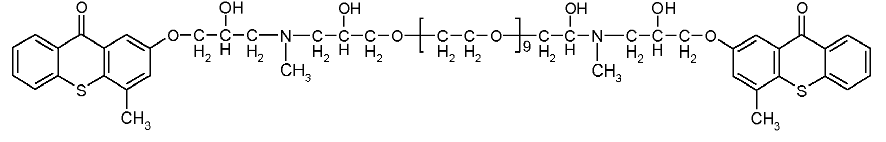 |
| ix) | 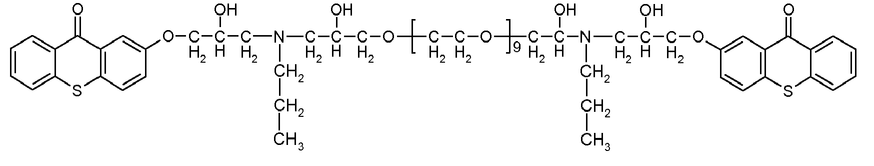 |
| x) | 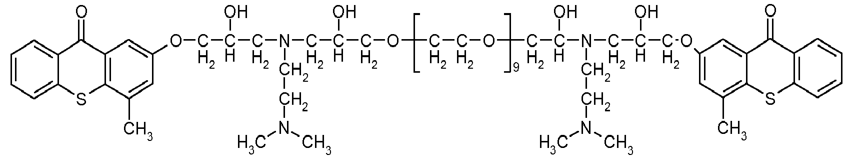 |
| xi) | 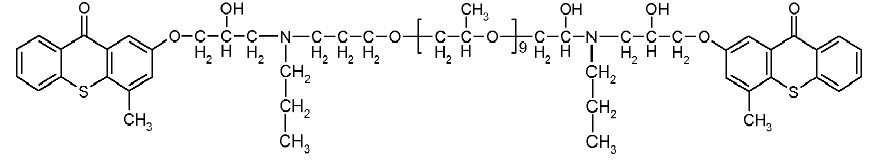 |
| xii) | 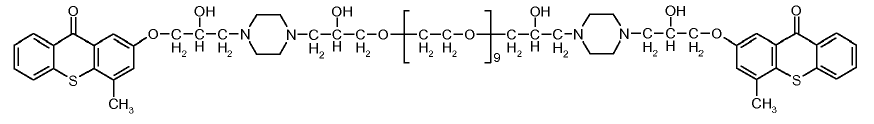 |

Another preferred group of compounds of the present invention is characterized by the following formula: 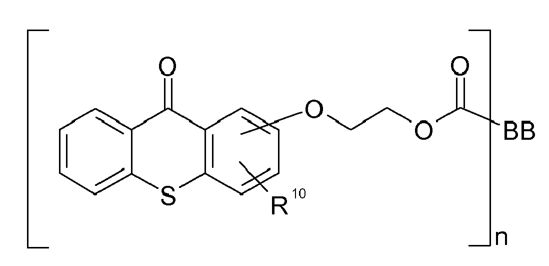 wherein
- R¹⁰: is H or a linear or branched C₁-₈ alkyl residue, preferably linear or branched C₁₋₄ alkyl residue, most preferably CH₃, or a halogen residue, preferably Cl;
- BB: is selected from the group consisting of 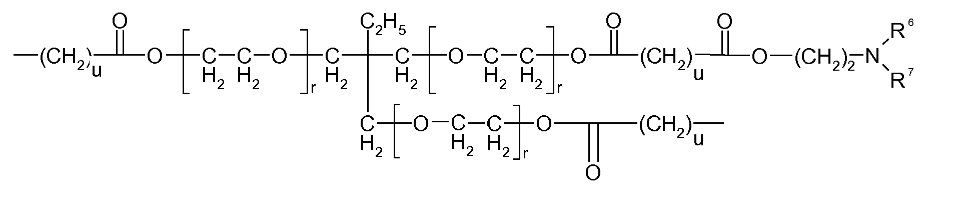 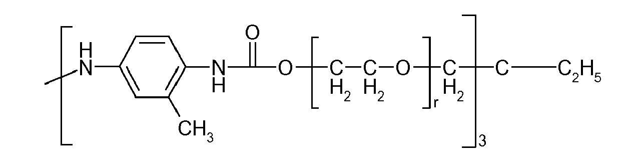 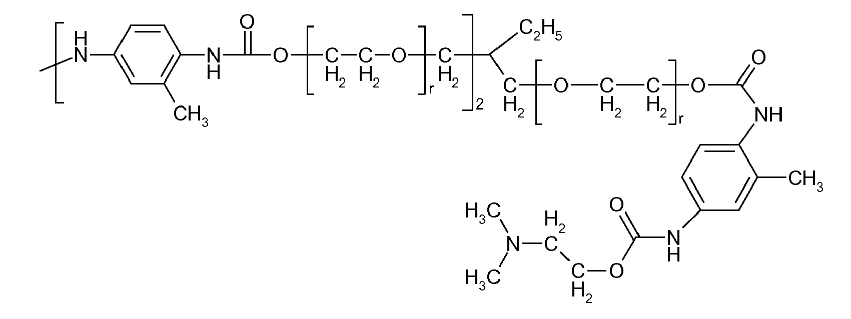 wherein r, R⁶, R⁷ are as defined above;
- N: is 2 or 3.

The PI-Sp unit to be used here is 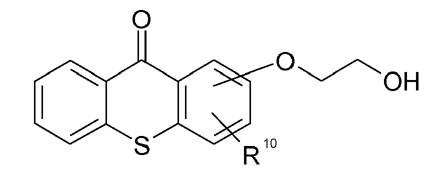 which synthesis has been described above. Preferably, the -OCH₂CH₂OH moiety is located in 2 position of the thioxanthone ring. However, in the case of chloro-substituted thioxanthones, it is preferred that the Cl residue is in 2 position and the -OCH₂CH₂OH moiety is in 4 position.

These compounds are characterized by either a urethane backbone or by a polyol backbone comprising terminating amine functionalities.

The urethane backbone can be synthesized by reacting the respective hydroxy thioxanthone derivative with a diisocyanate, followed by reaction with a respective polyol moiety. According to the present invention, in this preferred embodiment the polyol is a pentaerythritol-based polyol.

According to the present invention, a pentaerythritol-based polyol is a polyol comprising a quartenary carbon atom and having at least three hydroxy groups. A preferred example is polyol 3380:

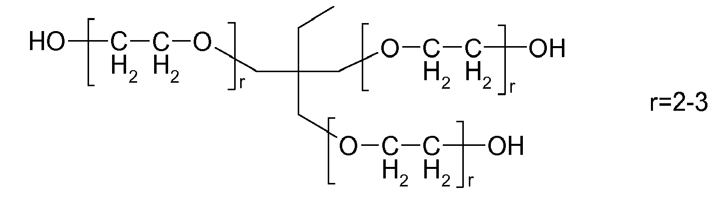

In the commercial product Polyol 3380, r is statistically 2.5.

Instead of attaching said polyol via a diisocyanate compound, said polyol may be attached to the PI-Sp moiety also via a dicarboxylic acid or a dicarboxylic acid derivative thereof, such as a dicarboxylic acid halide. For example, in order to obtain the first BB unit mentioned above in this embodiment, the PI-Sp unit is reacted with HOOC-(CH₂)₂-COOH, and subsequently with polyol 3380.

Preferred examples of compounds of this group are:

| | |
|---|---|
| xiii) | 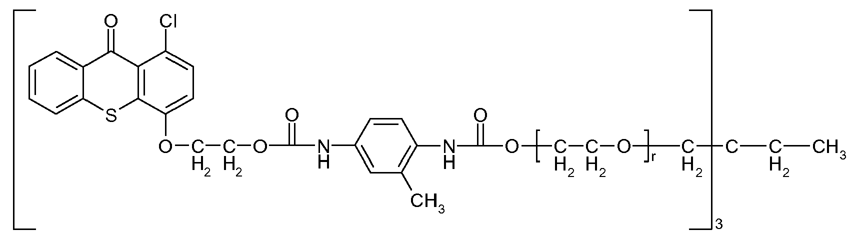 |
| xiv) | 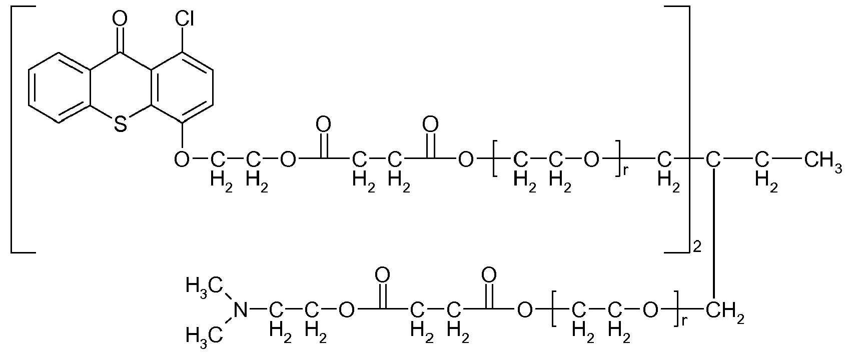 |
| xv) | 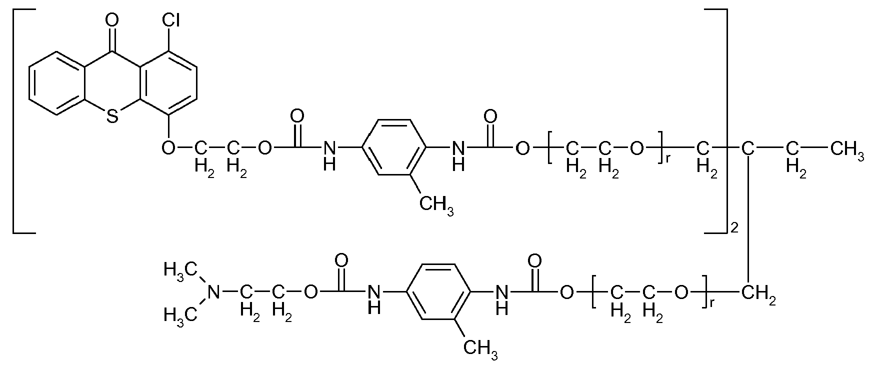 |
| xvi) | 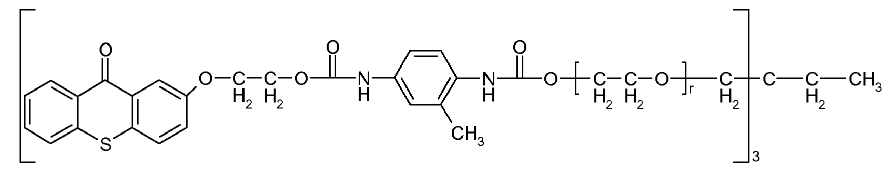 |
| xvii) | 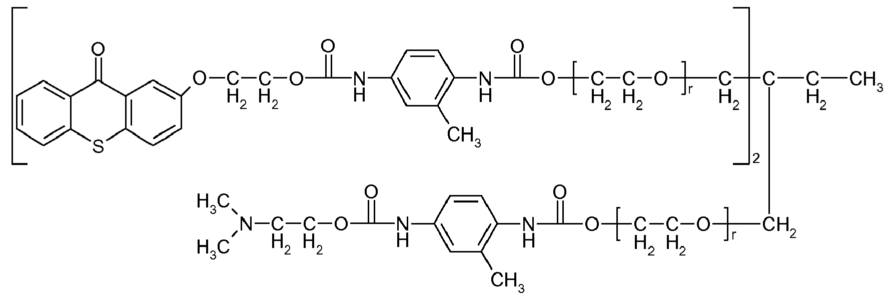 |

In the above formulae, r is as defined above. Depending on the ratio of the starting components, either one, two or all three hydroxy groups of the polyol 3380 may be reacted with the PI-Sp moiety. Remaining free hydroxy groups of the polyol 3380 may be reacted with terminal amino groups, as shown above.

Of course, also other polyols having three or more hydroxy groups can be used in a similar way as polyol 3380, in accordance with the present invention.

Another preferred group of compounds of the present invention is characterized by the formula 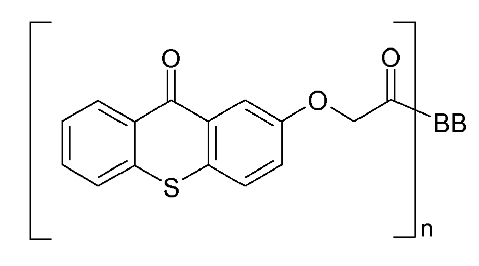 wherein
- BB: is selected from the group consisting of 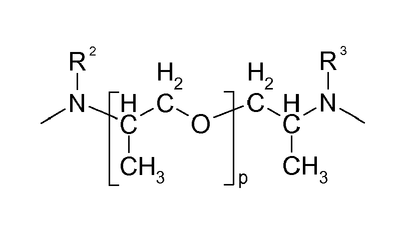 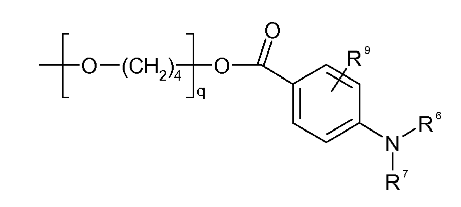 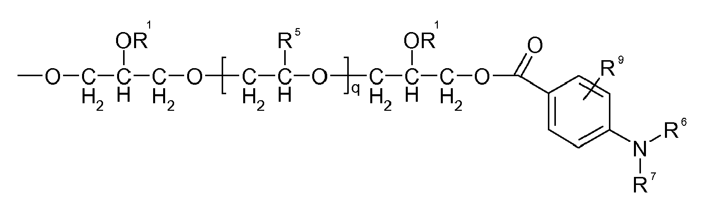 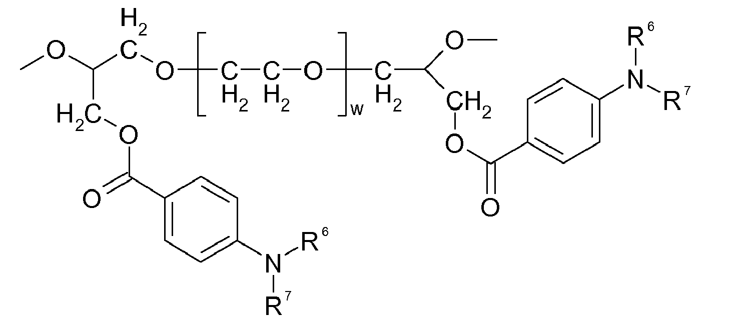 wherein
R¹ is H; 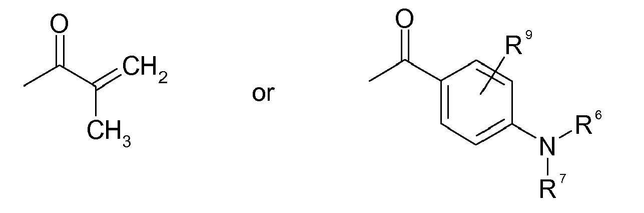 R² is H
p, q, r, R⁵, R⁶, R⁷ and R⁹ are as defined above
- n: is 1 or 2.

The PI-Sp unit to be used here is 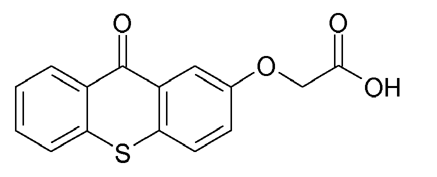 which synthesis has been described above.

The backbone units 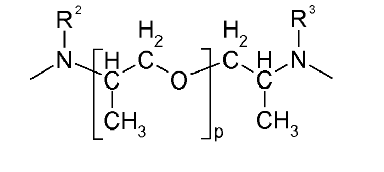 are derived from the commercially available polyoxypropylene amines such as Jeffamine D230 or Jeffamine D400.

The second backbone unit of the above list is obtained by reacting the respective PI-Sp unit with a polytetrahydrofurane, and subsequently with a 4-Amino benzoyl derivative.

The third backbone unit of the above list is obtained by reacting the respective PI-Sp unit with a polyoxyethylene diepoxide or polyoxypropylene diepoxide, and subsequently with a 4-Amino benzoyl derivative.

According to the present invention, in this preferred embodiment the backbone can be also made of a polyol which is a pentaerythritol-based polyol, as defined above. A preferred example is polyol 3380 which has been described above.

Preferred examples of compounds of this group are:

| | |
|---|---|
| xviii) | 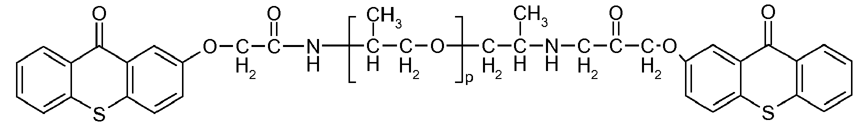 |
| xix) | 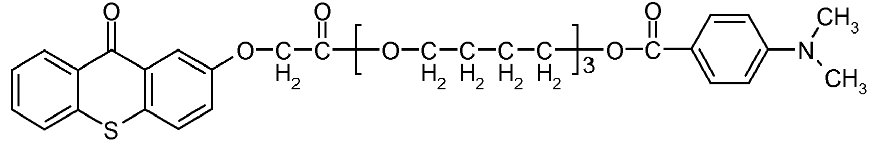 |
| xx) | 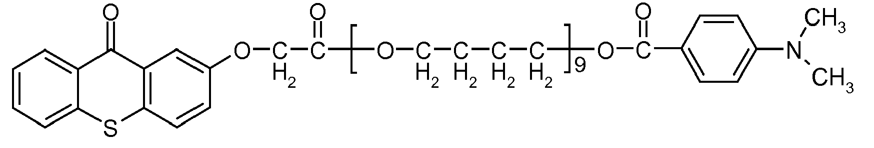 |
| xxi) | 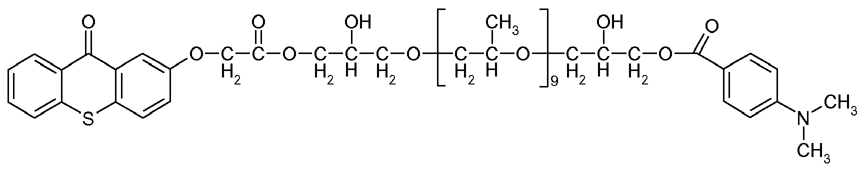 |
| xxii) | 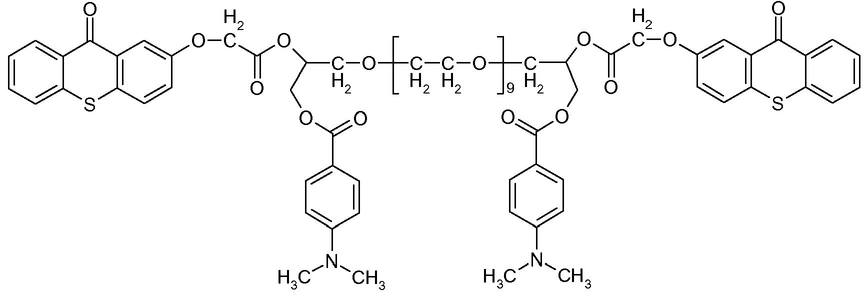 |

Another preferred group of compounds of the present invention is characterized by the formula 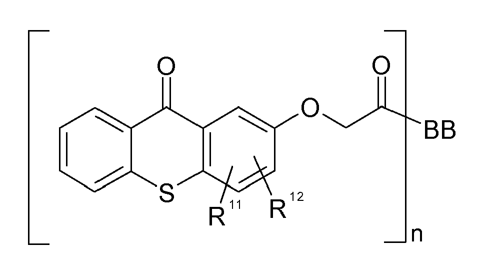 wherein
- R¹¹ and R¹²: are the same or different and denote H, a linear or branched C₁₋₈ alkyl, preferably lin- ear or branched C₁₋₄ alkyl, most preferably CH₃ or CH(CH₃)₂, linear or branched C₁₋₈ alkoxy, preferably linear or branched C₁₋₄ alkoxy, most preferably OCH₃, or halogen, preferably Cl;
- BB: is selected from the group consisting of 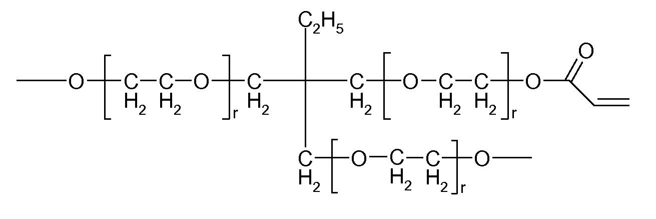 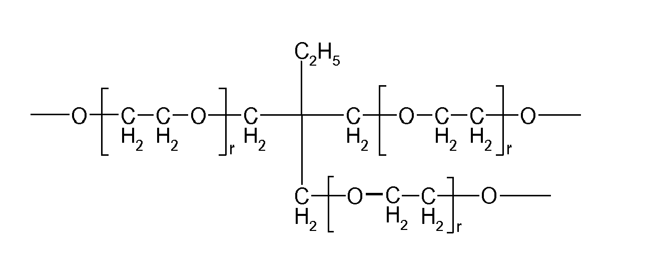 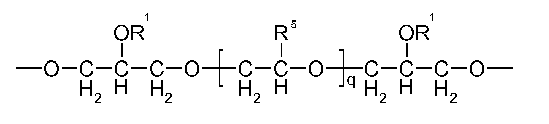 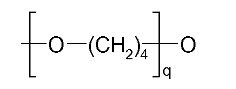 wherein R¹, R⁵, r and q are as defined above;
- n: is 2 or 3;
with the proviso that if R¹¹ and R¹² are H, BB is not

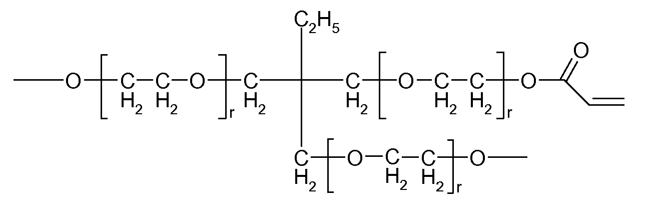

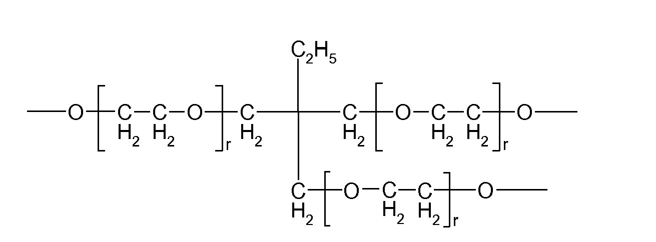

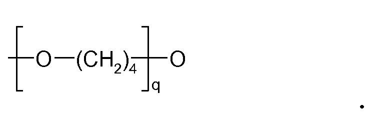

The PI-Sp unit to be used here is 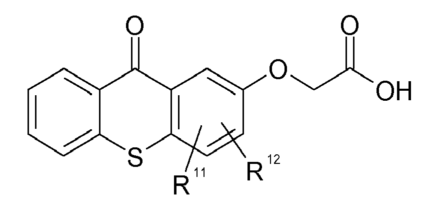 which synthesis has been described above.

In the first two backbones of the above list, the polyol is a pentaerythritol-based polyol as defined above, preferably polyol 3380. Depending on the ratio of the starting components, either one, two or all three hydroxy groups of the polyol 3380 may be reacted with the PI-Sp moiety. Remaining free hydroxy groups of the polyol 3380 may be reacted with terminal groups, for example an acrylic group, as shown above.

The third backbone unit of the above list is obtained by reacting the respective PI-Sp unit with a polyoxyethylene epoxide or polyoxypropylene epoxide.

The fourth backbone unit of the above list is obtained by reacting the respective PI-Sp unit with a polytetrahydrofurane.

Preferred examples of compounds of this group are:

| | |
|---|---|
| xxiii) | 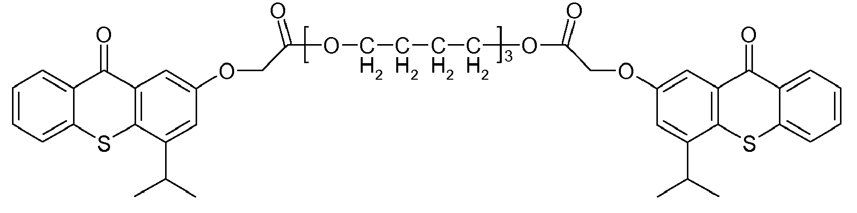 |
| xxiv) | 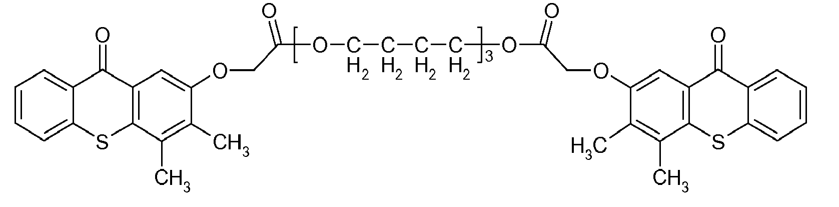 |
| xxv) | 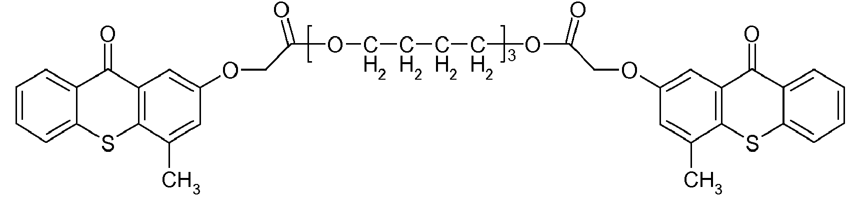 |
| xxvi) | 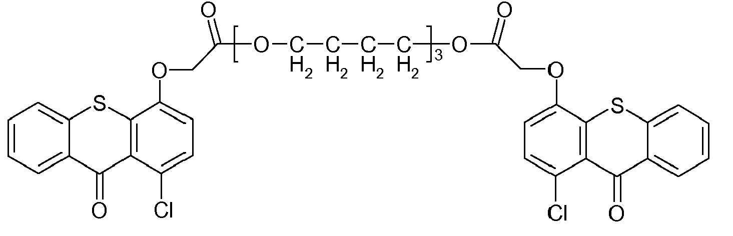 |
| xxvii) | 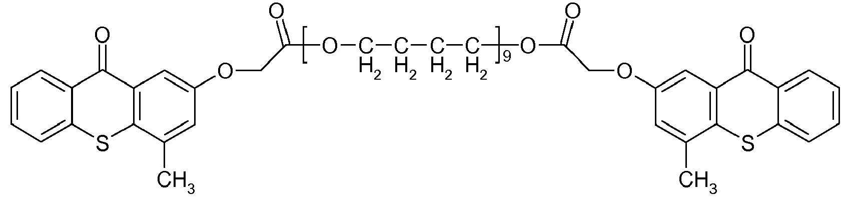 |
| xxviii) | 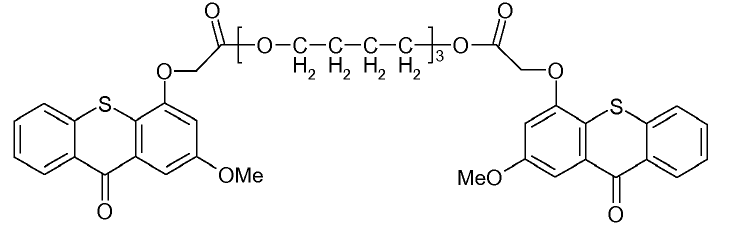 |
| xxix) | 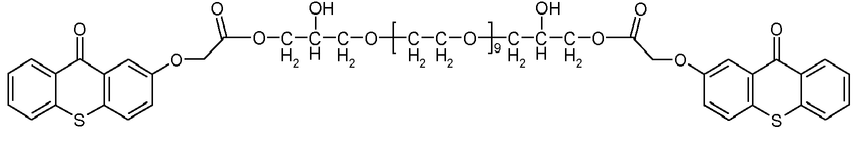 |

Another preferred group of compounds of the present invention is characterized by the formula 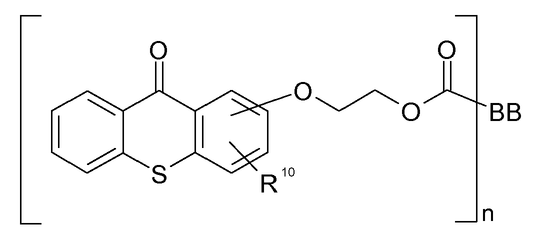 wherein
- R¹⁰: is H or Cl;
- BB: is selected from the group consisting of 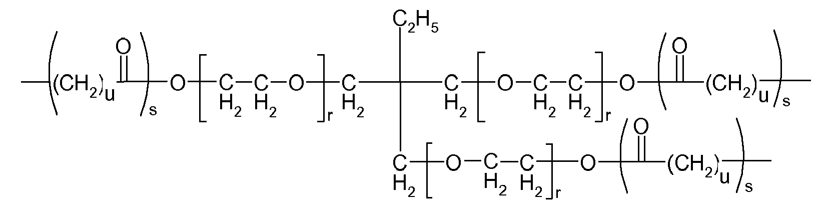 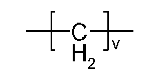 wherein
r is as defined above;
s is independently at each position 0 or 1;
v is an integer from 4 to 10, preferably 8;
- n: is 2 or 3.

The PI-Sp unit to be used here is 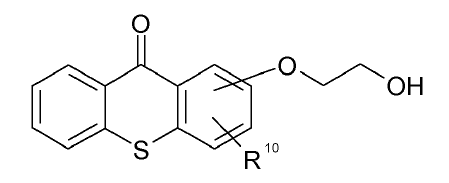 which synthesis has been described above.

According to the present invention, in this preferred embodiment the backbone is made of a polyol which is a pentaerythritol-based polyol, as defined above. A preferred example is polyol 3380 which has been described above.

Said polyol may be attached here to the PI-Sp moiety via a dicarboxylic acid or a dicarboxylic acid derivative thereof, such as a dicarboxylic acid halide. For example, the PI-Sp unit is reacted with HOOC-(CH₂)₂-COOH, and subsequently with polyol 3380. Depending on the ratio of the starting components, either one, two or all three hydroxy groups of the polyol 3380 may be reacted with the PI-Sp moiety. Remaining free hydroxy groups of the polyol 3380 may be reacted with terminal groups, as shown above.

In an alternative embodiment, the PI-Sp units may be reacted directly with a dicarboxylic acid or a dicarboxylic acid derivative thereof, such as a dicarboxylic acid halide, in order to obtain the products having the second backbone unit of the above list.

Preferred examples of compounds of this group are:

| | |
|---|---|
| xxx) | 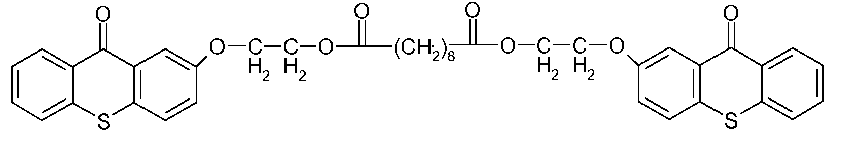 |
| xxxi) | 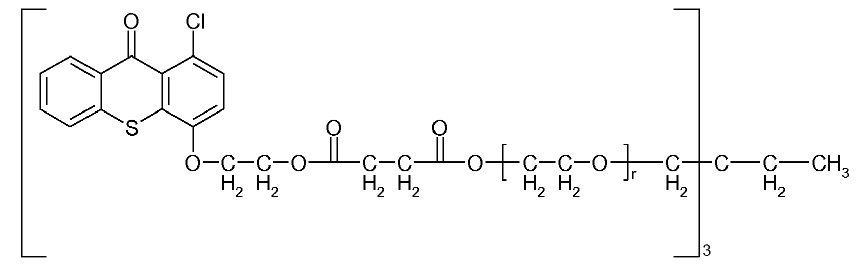 |

Another preferred group of compounds of the present invention is characterized by the formula 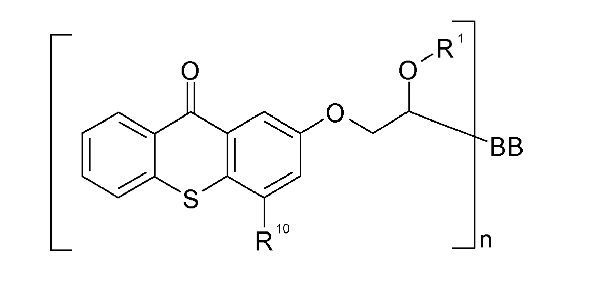 wherein
- R¹: is H or an acyl residue, preferably 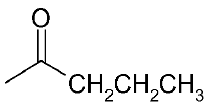
- R¹⁰: is H or a linear or branched C₁₋₈ alkyl residue, pref- erably linear or branched C₁₋₄ alkyl residue, most pref- erably CH₃;
- BB: is 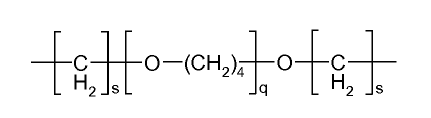 wherein
q is as defined above;
s is independently at each position 0 or 1;
- n: is 2.

The PI-Sp unit to be used here is 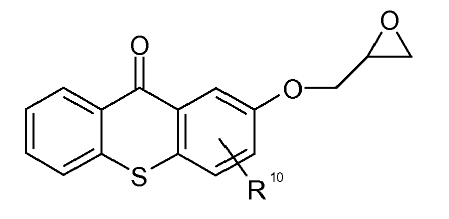 which synthesis has been described above.

The backbone unit is generated by reacting said PI-Sp unit with a polytetrathydrofurane derivative under reaction conditions which are well-known to the skilled person.

Subsequently, the hydroxy group generated through he above reaction may be converted into an ester moiety. Also the formation of esters from carboxylic acid derivatives and alcohols is well-known.

Preferred examples of compounds of this group are:

| | |
|---|---|
| xxxii) | 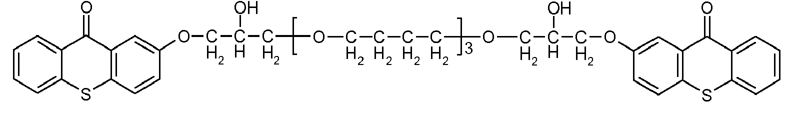 |
| xxxiii) | 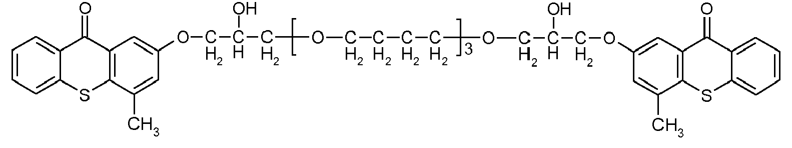 |
| xxxiv) | 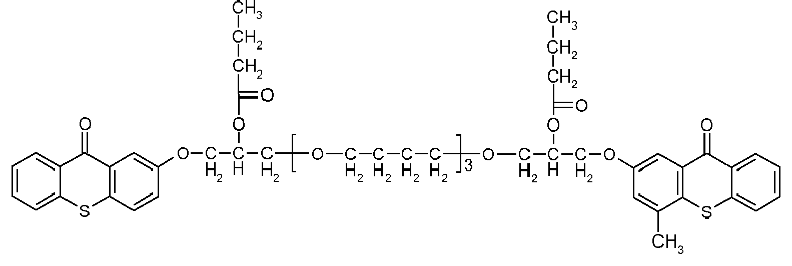 |

According to the present invention, also acridone derivatives proved useful as photoinitiators. A preferred group of acridone derivatives of the present invention is characterized by the formula 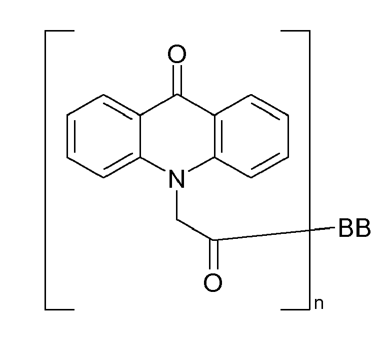 wherein BB is selected from the group consisting of 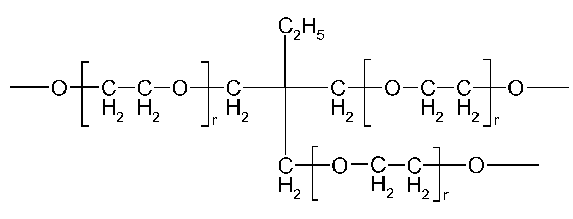 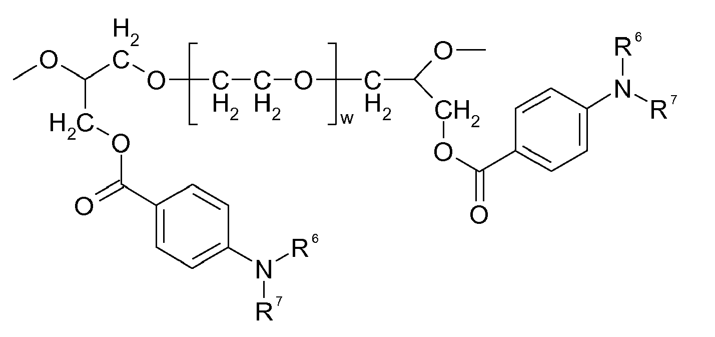 wherein
R6, R7, r and w are as defined above;
- n: is 2.

The PI-Sp unit of this embodiment may be prepared by the following scheme:

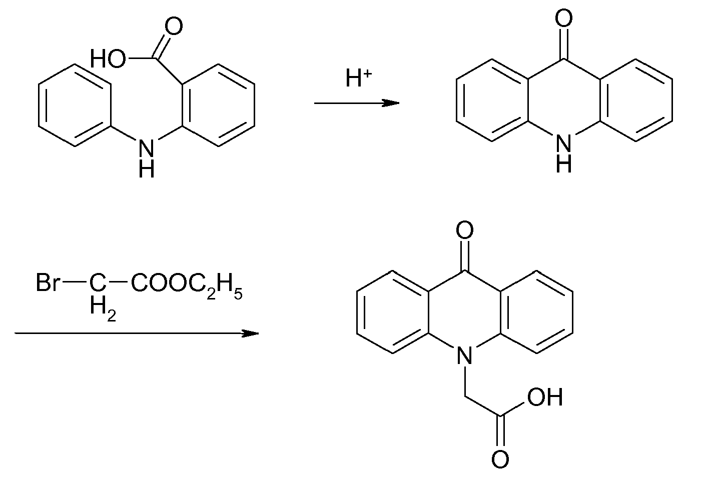

The ring closure reaction is carried out in the presence of an acid, such as polyphosphoric acid (PPA), under heating. Subsequently, the acridone is reacted with α-bromo acetic acid ester in the presence of a base, such as NaOH, under heating.

The thus obtained PI-Sp unit is further reacted with a pentaerythritol-based polyol such as polyol 3380, as defined above. Alternatively, said PI-Sp unit may also be reacted with a polyoxyalkylene epoxide such as a polyoxyethylene epoxide. The resulting hydroxy groups may subsequently be converted into esters, e.g. by reaction with a 4-aminobenzoyl derivative:

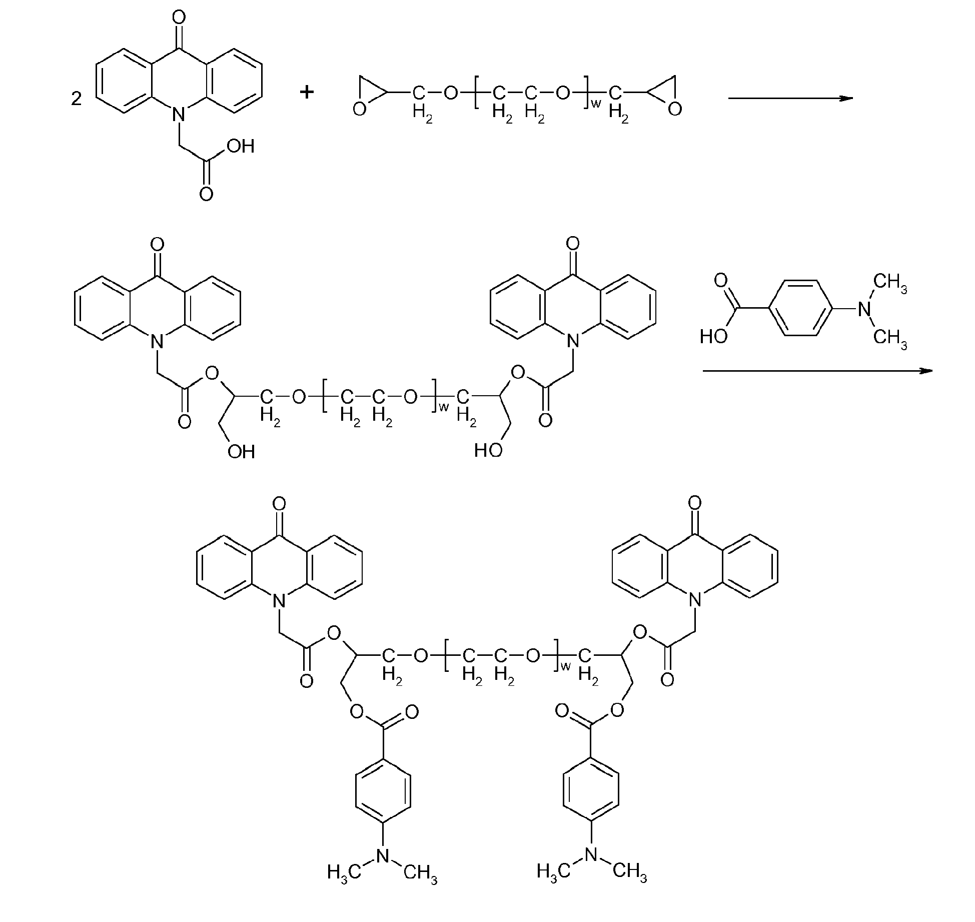

Preferred examples of compounds of this group are:

| | |
|---|---|
| xxxv) | 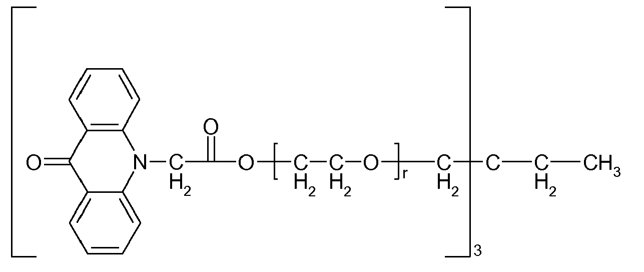 |
| xxxvi) | 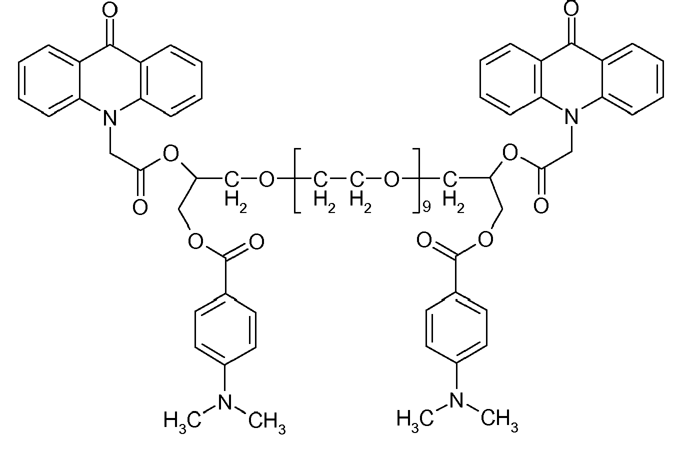 |

The above described photoinitiators may be added to conventional radiation-curable coating compositions. Such coating compositions are commonly known in the art and need not be reiterated here in detail.

A coating composition incorporating the photoinitiators of the present invention will normally comprise at least one radiation-curable monomer and/or oligomer, a photoinitiators of the present invention and optionally an additional reactive diluent. In the case of a printing ink, the composition will also contain a colorant, e. g. a pigment.

The radiation-curable monomer or oligomer is preferably an ethylenically unsaturated compound. Examples of suitable acrylate oligomers include aliphatic or aromatic urethane acrylates, polyether acrylates, polyester acrylates and epoxy acrylates (such as bisphenol A epoxy acrylate). Examples of suitable acrylate monomers include hexanediol diacrylate, trimethylolpropane triacrylate, di-trimethylolpropane tetra- acrylate, dipentaerythritol pentaacrylate, polyether acrylates, such as ethoxylated trimethylol propane triacrylate, glycerol propoxylate triacrylate, ethoxylated pentaerythritol tetraacrylate, and epoxy acrylates such as dianol diacrylate (= the diacrylate of 2, 2-bis [4- (2-hydroxyethoxy) phenyl] propane, Ebecryl 150 from UCB) and glycol diacrylates such as tripropylene glycol diacrylate.

It is a great benefit of the present invention that the majority of the photoinitiators described herein comprise an amine moiety within its molecule. Thus, the amount of additional synergist can be largely reduced or even for some applications completely be omitted. Therewith, any migration of said small molecule synergist from the final coating is largely reduced, leading to a reduction of environmental problems. However, for the use of photoinitiators of the present invention which do not comprise an amine moiety within the molecule, the compositions of the present intention preferably contain a synergist, such as an aminoacrylate or a dimethylaminobenzoic acid ester, as is well known in the art. Preferably the synergist will be a dimethylaminobenzoic acid ester in the case of a printing ink or an aminoacrylate in the case of a varnish. Some inks, such as those used in flexographic printing applications may contain both amine types.

The multi-functional initiators of formula (I) are especially suited for inks, especially printing inks, including lithographic inks. These typically comprise, as additional components to those referred to above, one or more of pigments, waxes, stabilisers, and flow aids, for example as described in"Printing Ink Manual", fourth edition, Leach R. H. et al. (eds.), Van Nostrand Reinhold, Wokingham, (1988), the disclosure of which is incorporated herein by reference. The photoinitiators of the present invention have shown a good compatibility with the other components of these compositions.

The present invention will now be further described with respect to non-limiting examples.

### Example 1: Synthesis of Thioxanthone derivatives

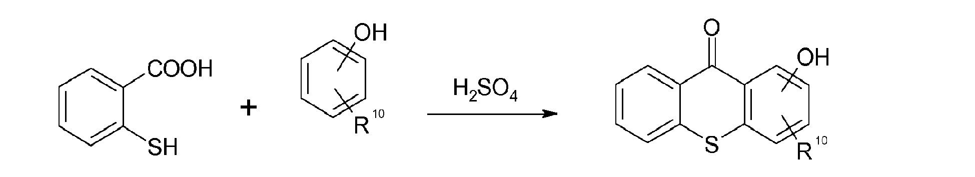

The general process for synthesis of HTX intermediates containing hydroxyl was carried out as follows: Thiosalycylic acid (0.1mol) was slowly added to 150ml of concentrated sulfuric acid, and the mixture was stirred for 5 min to ensure thorough mixing. Phenol derivatives (0.5mol) were added slowly to the stirred mixture over a period of 30 min. After the addition, the reaction mixture was stirred at room temperature for 1h and then at 80°C for 2h, after which it was left to stand at room temperature overnight. The resulting mixture was poured carefully with stirring into a 10-fold excess of boiling water, and was further boiled for 10 min. The solution was cooled and filtered. The residue was recrystallized from dioxane-water. The hydroxy thioxanthones were carefully characterized and confirmed by FT-IR and NMR.

By the above process, the following hydroxy thioxanthones were obtained:

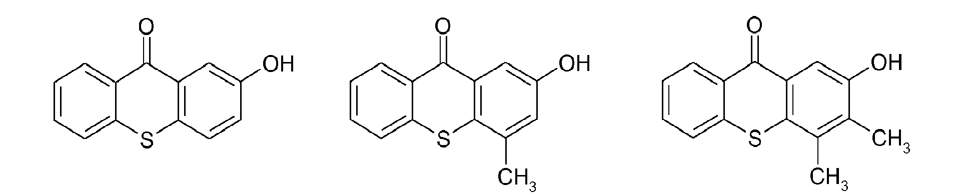

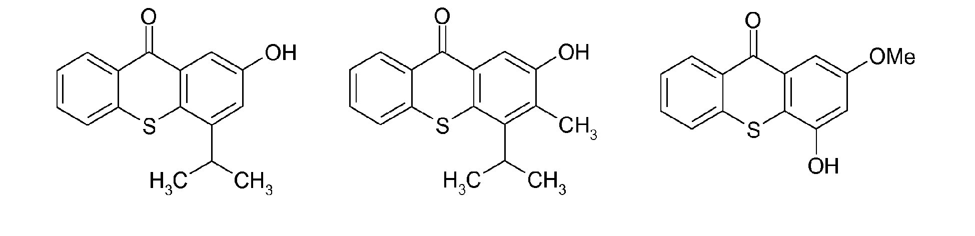

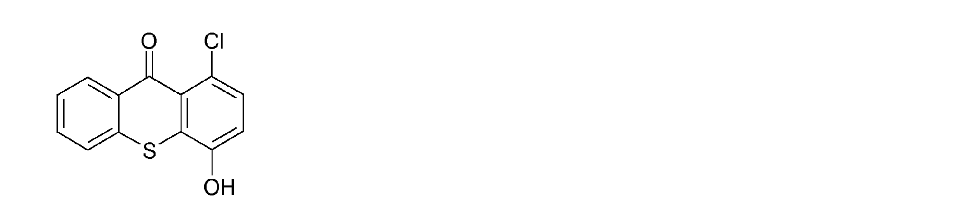

### Example 2: Synthesis of Acridone

2-Phenylamino benzoic acid was converted to acridone using polyphosphoric acid as acid component under heating at 110°C under conventional conditions.

### Example 3: Preparation of PI-SP units

### Example 3a: Acid-Thioxanthone intermediates

The synthesis of Acid-Thioxanthone intermediates is shown in an exemplary way on the synthesis of 4-Isopropyl-2-carboxymethyloxy-thioxanthone:

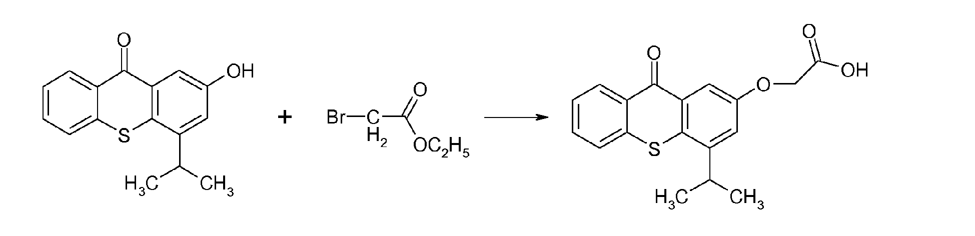

2.4g sodium hydroxide was refluxed in 40 ml tetrahyrofuran (THF) for 5 min. 4-Isopropyl-2-hydroxy thioxanthone (2.7g, 0.01 mol) was added and reflux was continued for 1 h, during which time the colour changed to red, indicating the formation of the sodium salt of 4-Isopropyl-2-hydroxy thioxanthone. Ethyl bromoacetate (3.51g, 0.021mol) was added and reflux was continued for 5 h, wherein the colour of the solution changed into yellow. After cooling to room temperature, 40 ml water was added, and the THF was distilled out to yield a clear red solution. The solution was refluxed for further 3 h in order to hydrolyse all the ester intermediate. The solution was then cooled to 50°C, and 40ml 1.0 M hydrochloric acid was added, causing the solid product to precipitate out. After refluxing for a further 10 min to be sure that all the sodium salt was converted to the free acid, the solution was cooled to room temperature and stirred for 2 h before filtering off the solid, washing with 40 ml water and drying in vacuum oven at 80°C. The yield was about 95%.

The other used Acid-Thioxanthone intermediates were obtained in the same way starting from the respective thioxanthone derivative.

### Example 3b: 2-Carboxymethyloxy thioxanthone

2-Carboxymethyloxy thioxanthone can be synthesized by a one-step reaction: Thiosalycylic acid (0.1mol) was slowly added to 150ml of concentrated sulfuric acid, and the mixture was stirred for 5min to ensure thorough mixing. Phenoxyl acetic acid (0.5mol) were added slowly to the stirred mixture over a period of 30 min. After the addition, the reaction mixture was stirred at room temperature for 1h and then at 80 for 2h, after which it was left to stand at room temperature overnight. The resulting mixture was poured carefully with stirring into a 10-fold excess of boiling water, and was further boiled for 10min. The solution was cooled and filtered. The residue was recrystallized from dioxane-water.

### Example 3c: Epoxy-Thioxanthone intermediates

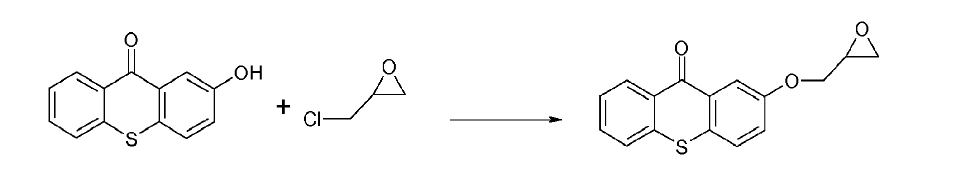

The general process for synthesis of epoxy-thioxanthone intermediates was as follows: A mixture of hydroxyl thioxanthone (0.03mol), epichlorohydrin(20ml), anhydrous potassium carbonate powder (6.5g), toluene (30ml), and polyethylene glycol (PEG-400, 0.5ml), was stirred for 2h at 80°C and then refluxed for 2h at 135°C. The organic layer was poured out after cooling, and the inorganic residue was washed with toluene until the solution became colourless. The combined solution was distilled to remove the solvent and excessive epichlorohydrin, and the residue was distributed between chloroform and water in a separating funnel. The chloroform solution was dried over anhydrous calcium chloride and evaporated. The crude product was recrystallized from ethanol-toluene (V/V=6/1).

The other used Epoxy-Thioxanthone intermediates were obtained in the same way starting from the respective thioxanthone derivative.

### Example 4: Preparation of Photoinitiators

### Example 4.1: Synthesis of

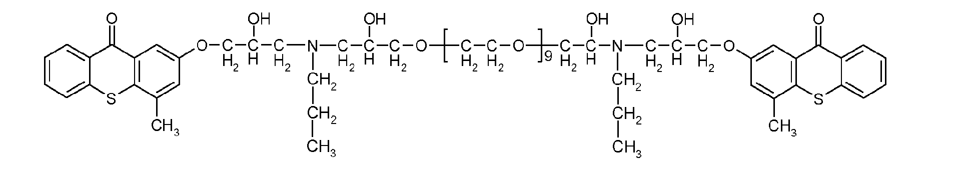

0.01mol 4-methyl-2-epoxymethyloxy thioxanthone (obtained in accordance with example 3c) and 0.04mol propylamine (PA) were dissolved in 20 ml chloroform, and the mixture was stirred at 40 °C for 12 hours. Unreacted PA and the solvent chloroform were removed by rotary evaporation, to obtain 4-methyl-2-(1-propyl-amino-2-hydroxy)-propoxy thioxanthone (4M2ETX-PA). 0.005mol PEO526 (Polyoxyethylene diepoxide) and 40 ml ethanol were added into flask containing 4M2ETX-PA. The mixture was refluxed at 80 °C for 8 hours under N₂ atmosphere. The solvent ethanol was removed by rotary evaporation, to obtain the title compound.

In a similar manner, by using other amine compounds such as piperazine further photoinitiators comprising amine moieties within the backbone could be generated.

In the case of using polymeric amines like Jeffamines, it was not necessary to add an polyalkoxy compound.

### Example 4.2: Synthesis of

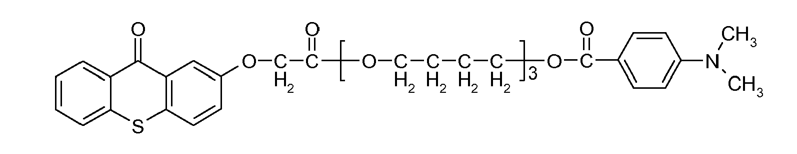

2-Carboxymethyloxy thioxanthone (2.88g, 0.01 mol, obtained in accordance with example 3b), p-THF 650 (6.50g, 0.01 mol), 4-dimethylaminobenzoic acid (2g, 0.012 mol), 0.3g p-toluene sulphonic acid and 100 ml xylene were added into a three-necked flask equipped with a nitrogen inlet, and a Dean-Stark trap. The mixture solution was azeotropically distilled at 150 °C for 12 h, and then xylene was distilled off. The mixture was heated to 200 °C for 5h and then cooled to 80°C. Subsequently, 100 ml toluene was added with stirring. The solution was cooled to room temperature, then filtered, and washed twice with 100 ml 0.25M sodium hydroxide and twice with a 100 ml water. The organic phase was dried over anhydrous magnesium sulphate and all solvent was removed using a rotary evaporator to yield a red oil. The yield was about 86%.

In a similar manner, by using other polyalkoxy compounds compounds such as polyoxyethylene diepoxide or poyloxypropylen diepoxide, further photoinitiators comprising terminal amine moieties could be generated.

In the case of using polymeric amines like Jeffamines, it was not necessary to add an polyalkoxy compound.

### Example 4.3: Synthesis of

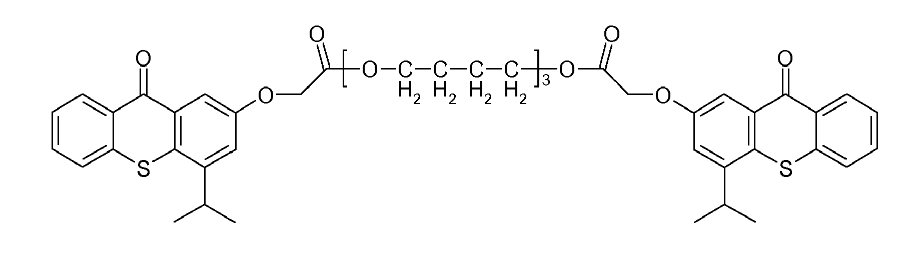

4-Isopropyl-2-carboxymethyloxy-thioxanthone (3.4g, 0.0103 mol, obtained in accordance with example 3a), p-THF 250 (1.25g, 0.005 mol), 0.3g p-toluene sulphonic acid and 80 ml toluene were added into a three-necked flask equipped with a nitrogen inlet, and a Dean-Stark trap. The mixture solution was azeotropically distilled for 10 h, and then cooled, filtered and washed twice with 100 ml 0.25M sodium hydroxide and twice with a 100 ml water. The organic phase was dried over anhydrous magnesium sulphate and all solvent was removed on a rotary evaporator to yield a red oil. The yield was about 91%.

### Example 4.4: Synthesis of

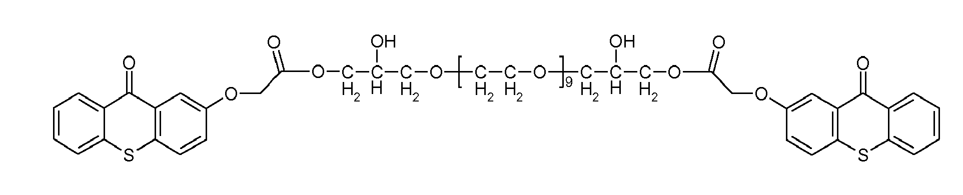

0.2 mol 2-Carboxymethyloxy thioxanthone (obtained in accordance with example 3b) and 0.1 mol PEO 526 (Polyoxyethylene diepoxide) were added into a flask. The mixture was heated to 150 ^{°}C for 12 h, and then to 180 ^{°}C for 6 h, to obtain the title compound. The yield was about 100%.

It is understood that the other compounds of the present invention may be prepared in a similar manner by using respective starting or intermediate materials. Optional slight variations of the reaction conditions are within the common routine of the skilled person.

Derivatization reactions (such as esterifying free hydroxy groups or amidifying free amino groups) can be carried out by standard procedures which need not be described in detail here.

### Example 5: Preparation of a printing ink

### a) Ink with amine-containing photoinitiator

A printing ink was prepared by mixing, in a conventional manner, the following components:

| **Component** | **Wt.-%** |
|---|---|
| Ebecryl 657 (Polyester Acrylate) | 30 |
| Ebecryl 1608 (Epoxy Acrylate) | 22,3 |
| TMPTA (Monomer) | 20 |
| Photoinitiator (example 4.2) | 8 |
| Speedcure EDB (Ethyl Di methyl amino benzoate, synergist) | 1,2 |
| Florstab UV 1 (Stabiliser) | 0,5 |
| Ciba Irgalite GLO (Pigment, PB 15:3) | 18 |

The ink exhibited good curing properties without the need of using an additional amine synergist.

### b) Ink without amine-containing photoinitiator

A printing ink was prepared by mixing, in a conventional manner, the following components:

| **Component** | **Wt.-%** |
|---|---|
| Ebecryl 657 (Polyester Acrylate) | 30 |
| Ebecryl 1608 (Epoxy Acrylate) | 23,5 |
| TMPTA (Monomer) | 20 |
| Photoinitiator (example 4.4) | 4 |
| Speedcure EDB (Ethyl Di methyl amino benzoate, synergist) | 4 |
| Florstab UV 1 (Stabiliser) | 0,5 |
| Ciba Irgalite GLO (Pigment, PB 15:3) | 18 |

The ink exhibited good curing properties.

### Test procedures

### a) Film alcohol resistance test

On a plastic substrate, a standard stample is provided next to a sample of a coating composition of the present invention. The curing is carried out with a mercury bulb (160W/cm , speed of conveyer 30m/min). A side by side draw down is carried out on a plastic film, including the standard and the test ink. With a cotton soaked with alcohol, the ink film is treated until disappearance. The number of double rubs which are necessary in this respect give the level of curing.

### b) Curing limit

A ink of the present invention is printed on a plastic substrate with a Little Joe or PrüfBau machine, at 1,50 g/m². Several pieces of the print are cut. Each piece is passed in the UV dryer with a conveyor speed decreasing step by step at each sample. It is evaluated with a thumb turn whether the film is cured or not. If not, the speed is reduced, and another piece of print is passed and evaluated again in curing. As the speeds is reduced, the irradiation becomes sufficient to have the ink fully cured, and the thumb does not leave a mark on the print anymore. The speed of the dryer is then recorded, and taken as the "curing limit" (m/min). The higher the figure, the higher the reactivity of the ink.

In the following table, the results of the curing limit test for several inks of the present invention comprising various photoinititiators of the present invention as compared to the reference product Omnipol TX (WO 03/033492, ex. 3) are given. The inks were prepared in accordance with example 5 (the ink according to comparative example 1 (C1) was prepared according to example 5b, using 3 wt.-% Omnipol TX and accordingly 24,5 wt.-% Ebecryl 1608):

| Ink no. | Photoinitiator in ink | Curing limit (m/min) |
|---|---|---|
| C1 | 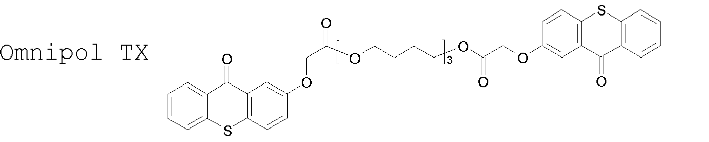 | 130 |
| 1 | 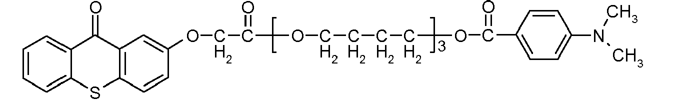 | 150 |
| 2 | 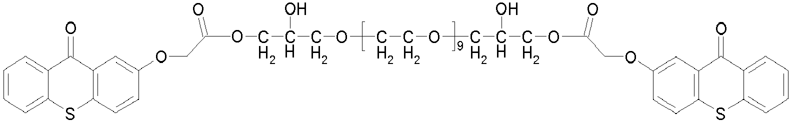 | 140 |

It was shown that the inks of the present invention showed a comparable or even better curing limit value than the reference example Omnipol TX. Moreover, in the case of example 1 the amount of additional synergist could be significantly reduced, which lowered migration problems caused by the synergist.

### c) Blocking test

Drawdown of an ink of the present invention versus a standard is conducted on a porous paper (paper printer). The curing parameter is : 1 pass 120 m/min 1 lamp min power.The print is maintained versus a blank piece of the porous paper under pressure (10 tons) at room temperature for 30 seconds. The results are expressed in function of the blocking scale and provides an assessment of the surface cure.

## Claims

1. Photoinitiators of the formula (I)
(PI-Sp)ₙ-BB (I)
wherein
PI is a thioxanthone or acridone moiety optionally com- prising additional substituents further to the Sp moi- ety
Sp is a spacer unit which, if PI is a thioxanthone moiety, is selected from the group consisting of 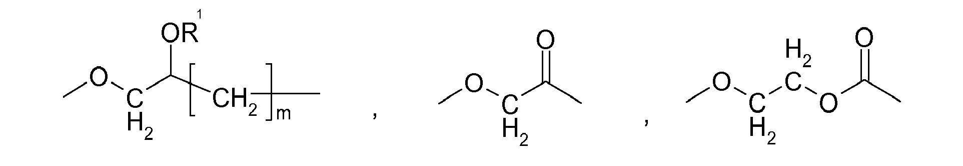 or, if PI is an acridone moiety, is 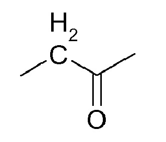 wherein
R1 is H, an optionally substituted linear or branched C₁₋₈ alkyl residue, an optionally substituted C₃₋₁₀ cycloalkyl residue or an acyl residue;
m is 0 or 1
BB is a backbone moiety selected from the group consisting of optionally substituted heteroaliphatic or heteroali- cyclic polyoxyethylene amines or polyoxypropylene amines, optionally substituted urethanes, optionally substituted pentaerythritol-based polyols, optionally substituted polyoxyethylene oxides and optionally substituted polyoxypropylene oxides; and
n is 1, 2 or 3;
with the proviso that if Sp is 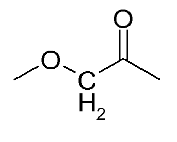 and PI is thioxanthone and does not comprise additional substituents besides Sp, then BB is not
a moiety from the group consisting of [O(CHR²CHR¹)ₐ]_{y}-Q,-[O(CH₂)_{b}CO]y-Q, or-[O(CH₂)_{b}CO_{(y-1)}[O(CHR²CHR¹)ₐ]-Q, where one of R¹ and R² represents a hydrogen atom and the other represents a hydrogen atom, a methyl group or an ethyl group; a is a number from 1 to 2; b is a number from 4 to 5; Q is a residue of a polyhydroxy compound having 2 to 6 hydroxy groups; y is a number from 3 to 10.

2. Photoinitiators according to claim 1, wherein the backbone moiety BB is selected from the group consisting of 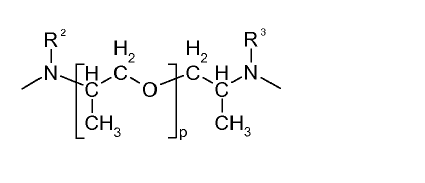 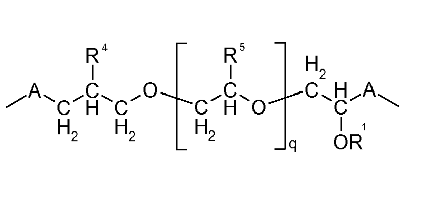 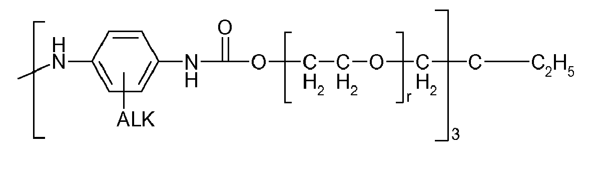 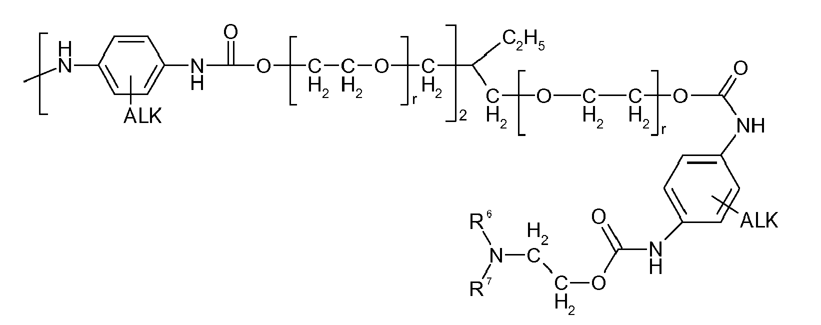 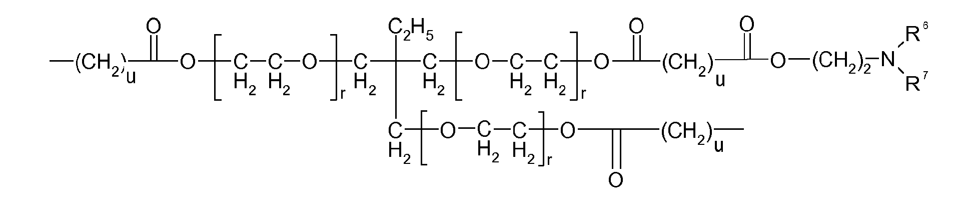 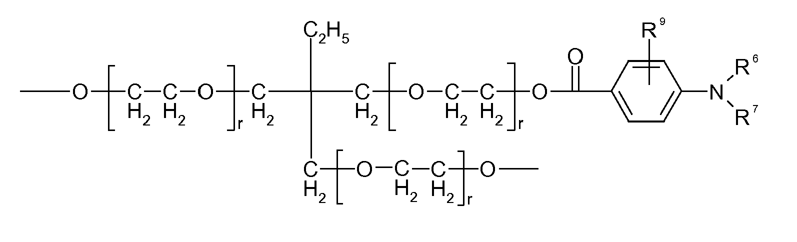 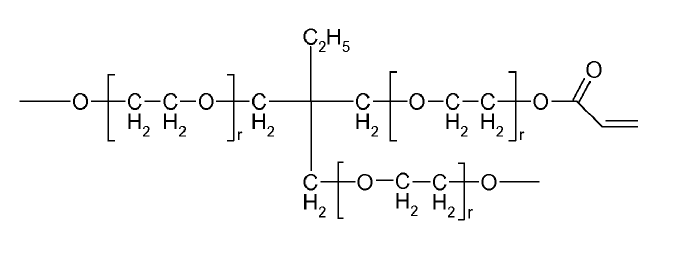 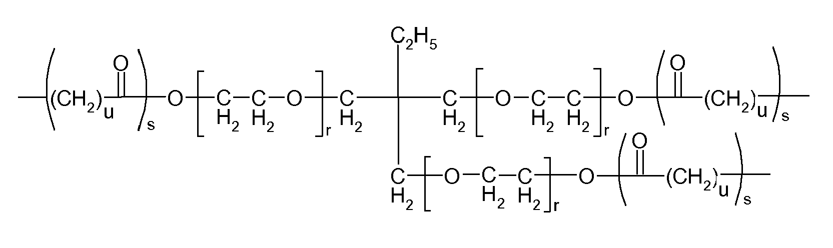 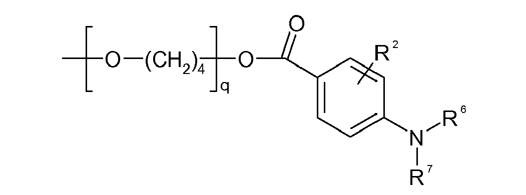 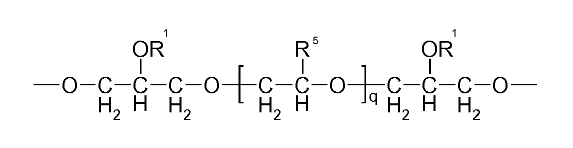 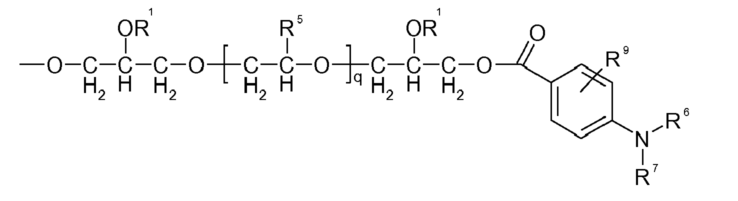 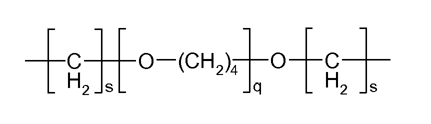 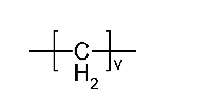 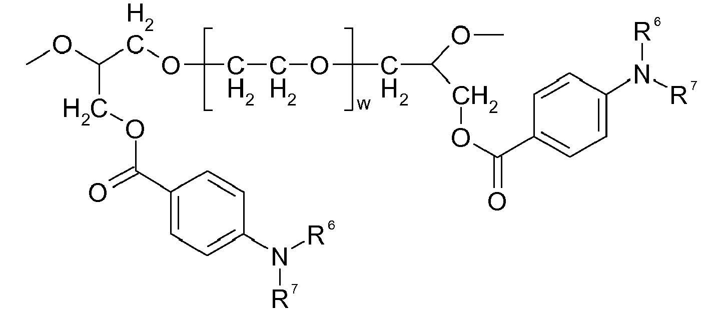 wherein
R¹ is as defined above;
R² and R³ are the same or different and denote H, an op- tionally substituted linear or branched C₁₋₈ alkyl residue, an optionally substituted C₃₋₁₀ cycloal- kyl residue, an acyl residue, or an optionally substituted C₁₋₈ alkylamine residue;
R⁴ is H or Hydroxy or Alkoxy
R⁵ is H or CH₃
R⁶ and R⁷ are the same or different and denote H, an op- tionally substituted linear or branched C₁₋₈ alkyl residue, or an optionally substituted C₃₋₁₀ cycloalkyl residue;
A is NR⁸ or an optionally substituted piperazine moiety, wherein
R⁸ is H, an optionally substituted linear or branched C₁₋₈ alkyl residue, an optionally substituted C₃₋₁₀ cycloalkyl residue, an acyl residue, or an optionally substituted C₁₋₈ al- kylamine residue;
ALK is an optionally substituted linear or branched C₁₋₈ alkyl residue;
R⁹ is H or a linear or branched C₁₋₈ alkyl residue, preferably linear or branched C₁₋₄ alkyl residue
p is an integer from 1 to 10, preferably from 3 to 6;
q is an integer from 1 to 15, preferably from 3 to 10;
r is in the range from 2 to 3;
s is independently at each position 0 or 1;
u is an integer from 1 to 6;
v is an integer from 4 to 15;
w is an integer from 2 to 15, preferably from 3 to 10.

3. Photoinitiators according to claim 1 or 2, wherein the SP moiety is linked to the 2 position of the PI thioxanthone moiety.

4. Photoinitiators according to one of claims 1 to 3, wherein the moiety PI is substituted with at least one residue selected from the group consisting of linear or branched C₁₋₈ alkyl, preferably linear or branched C₁₋₄ alkyl, linear or branched C₁₋₈ alkoxy, preferably linear or branched C₁₋₄ alkoxy, or halogen, preferably Cl.

5. Photoinitators according to claim 4, wherein in 4-position of the PI moiety there is a linear or branched C₁₋₈ alkyl residue, preferably linear or branched C₁₋₄ alkyl residue.

6. Photoinitiators according to one of claims 1 to 5, having the formula 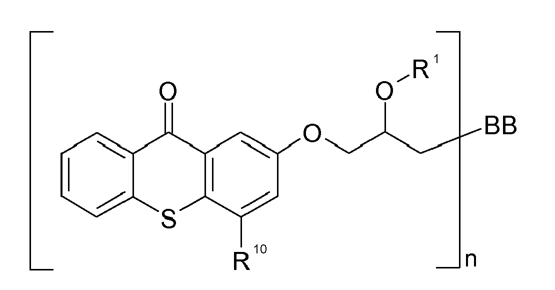 wherein
R¹ is H, 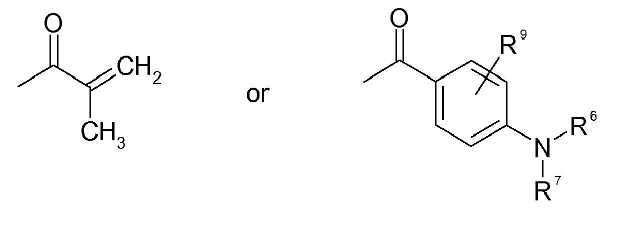 wherein
R⁶ and R⁷ are as defined above, preferably both CH₃;
R⁹ is as defined above, preferably H or CH₃.
R¹⁰ is H or a linear or branched C₁₋₈ alkyl residue, pref- erably linear or branched C₁₋₄ alkyl residue, most pref- erably CH₃;
BB is selected from the group consisting of 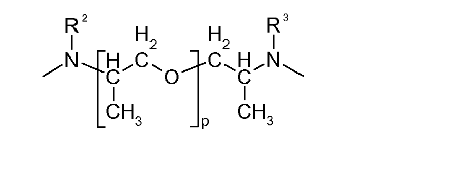 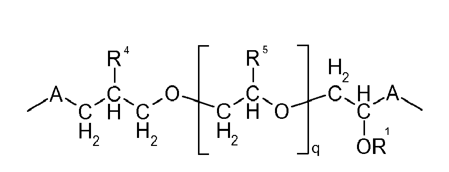 wherein
R¹ is H; 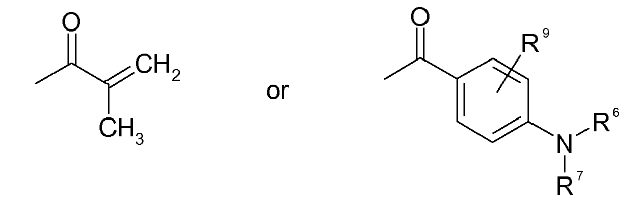
R² and R³ are the same or different and denote H, a linear or branched C₁₋₈ alkyl residue, preferably linear or branched C₁₋₄ alkyl residue, a linear or branched C₁₋₈ alkylamine residue, preferably linear or branched C₁₋₄ alkylamine residue, 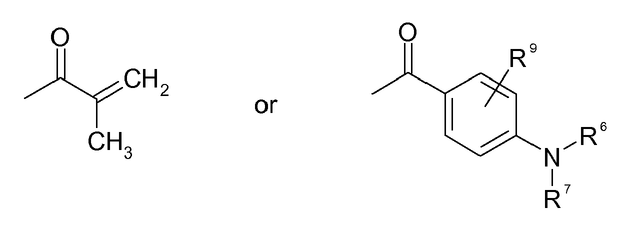 wherein R⁶, R⁷ and R⁹ are as defined above;
p is an integer from 1 to 10, preferably from 3 to 6, most preferably 3 or 5;
q is an integer from 1 to 15, preferably from 3 to 10, most preferably 9;
A is NR⁸ or an optionally substituted piperazine or piperazine methylene moiety, wherein R⁸ is H, a linear or branched C₁₋₈ alkyl residue, preferably linear or branched C₁₋₄ alkyl residue, a linear or branched C₁₋₈ alkylamine residue, preferably linear or branched C₁₋₄ alkylamine residue, 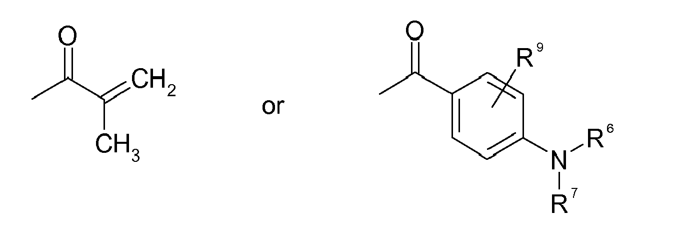 wherein R⁶, R⁷ and R¹¹ are as defined above;
R⁴ is H or Hydroxy;
R⁵ is H or CH₃; and
n is 2.

7. Photoinitiators according to one of claims 1 to 5, having the formula 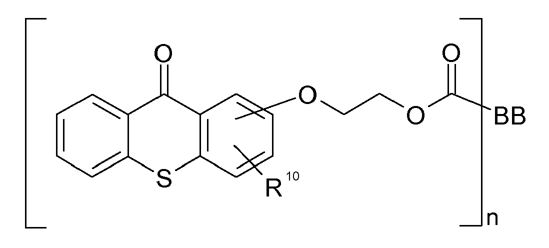 wherein
R¹⁰ is H or a linear or branched C₁₋₈ alkyl residue, pref- erably linear or branched C₁₋₄ alkyl residue, most pref- erably CH₃, or a halogen residue, preferably Cl;
BB is selected from the group consisting of 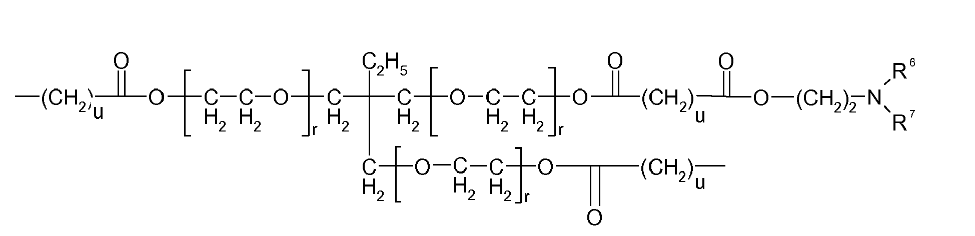 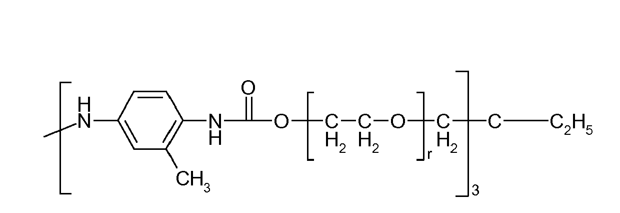 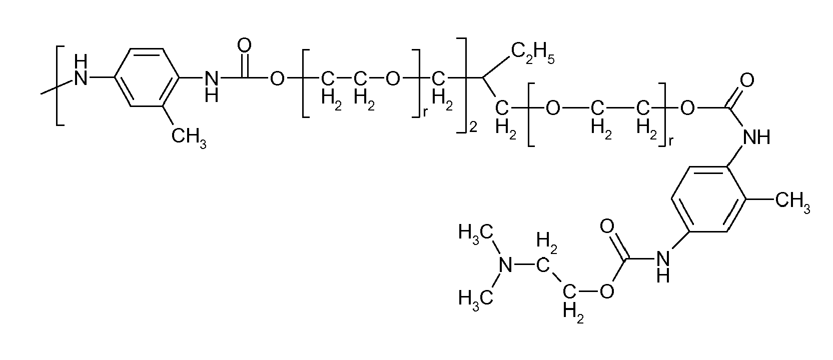 wherein r,u, R⁶, R⁷ are as defined above;
n is 2 or 3.

8. Photoinitiators according to one of claims 1 to 5, having the formula 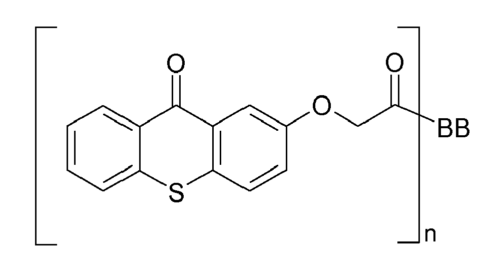 wherein
BB is selected from the group consisting of 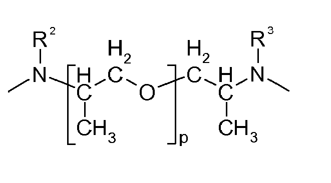 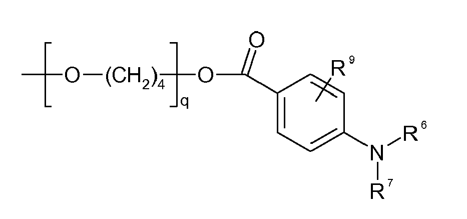 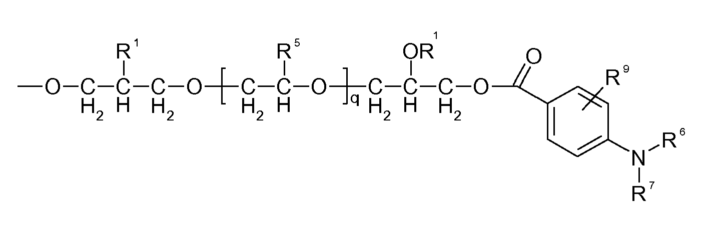 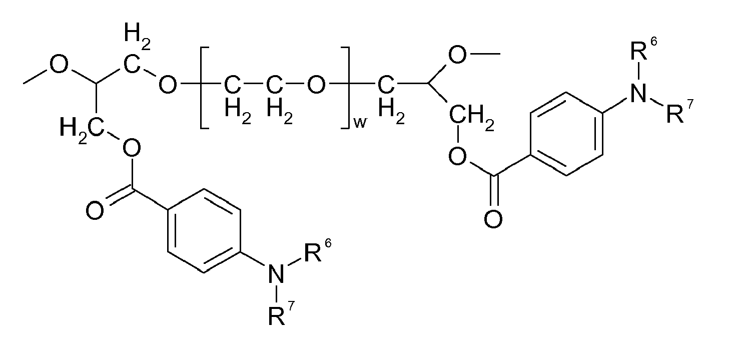 wherein
R¹ is H; 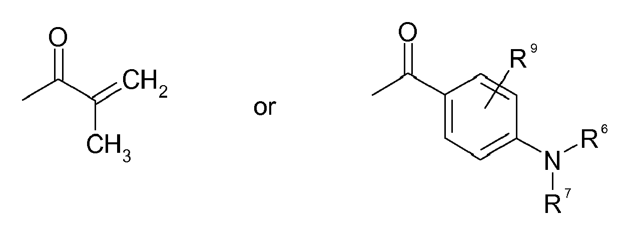
R² is H
p, q, r, w, R⁵, R⁶, R⁷ and R⁹ are as defined above
n is 1 or 2.

9. Photoinitiators according to one of claims 1 to 5, having the formula 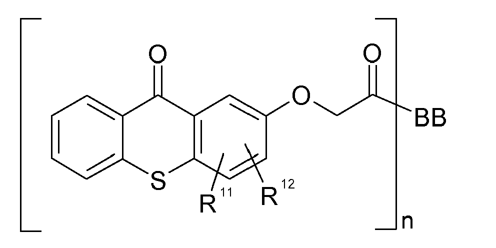 wherein
R¹¹ and R¹² are the same or different and denote H, a linear or branched C₁₋₈ alkyl, preferably lin- ear or branched C₁₋₄ alkyl, most preferably CH₃ or CH(CH₃)₂, linear or branched C₁₋₈ alkoxy, preferably linear or branched C₁₋₄ alkoxy, most preferably OCH₃, or halogen, preferably Cl;
BB is selected from the group consisting of 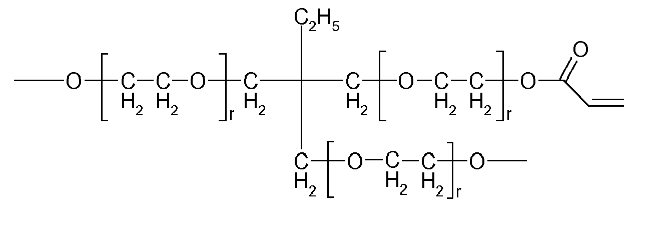 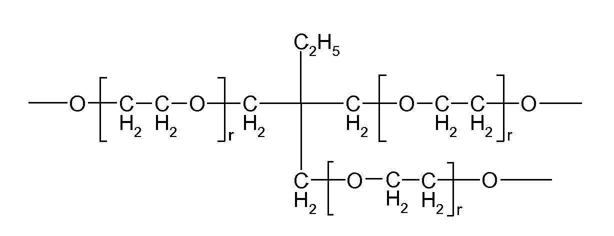 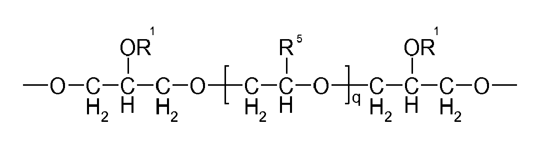 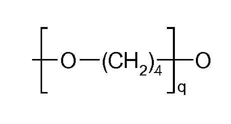
wherein R¹, R⁵, r and q are as defined above;
n is 2 or 3;
with the proviso that if R¹¹ and R¹² are H, BB is not

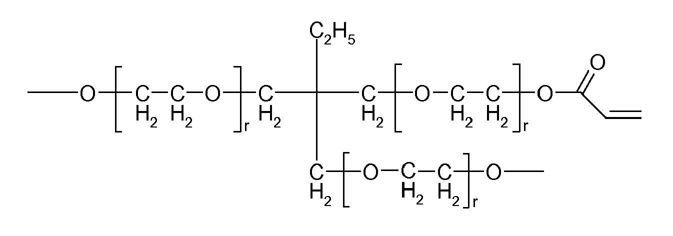

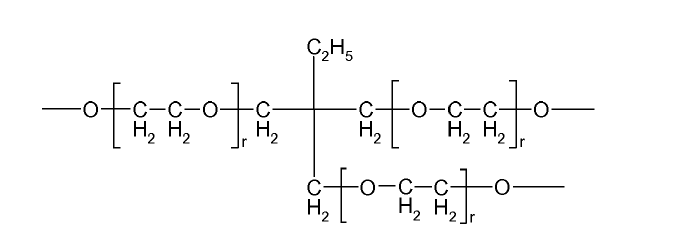

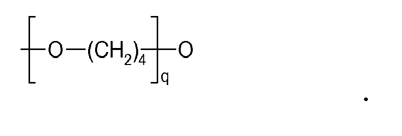

10. Photoinitiators according to one of claims 1 to 5, having the formula 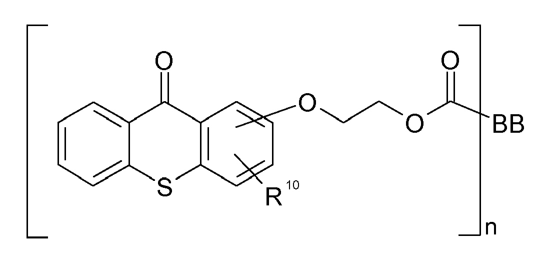 wherein
R¹⁰ is H or Cl;
BB is selected from the group consisting of 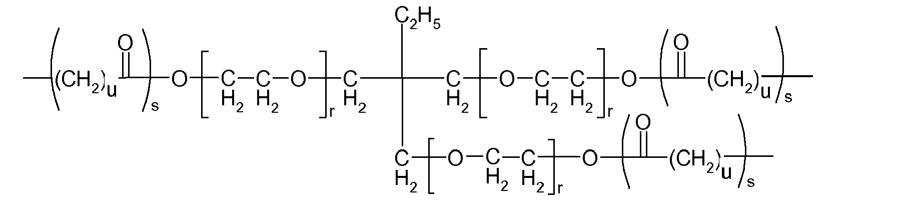 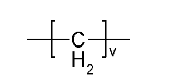 wherein
r is as defined above;
s is independently at each position 0 or 1;
v is an integer from 4 to 10, preferably 8;
n is 2 or 3.

11. Photoinitiators according to one of claims 1 to 5, having the formula 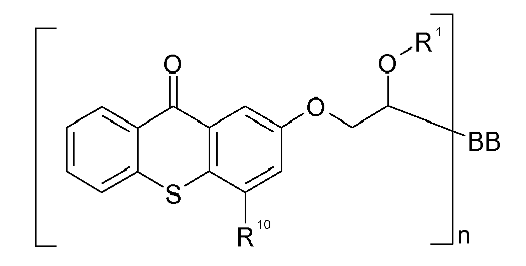 wherein
R¹ is H or an acyl residue, preferably 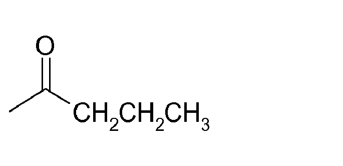
R¹⁰ is H or a linear or branched C₁₋₈ alkyl residue, pref- erably linear or branched C₁₋₄ alkyl residue, most pref- erably CH₃;
BB is 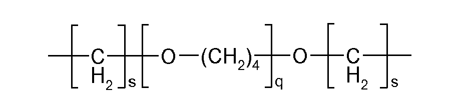 wherein
q is as defined above;
s is independently at each position 0 or 1;
n is 2.

12. Photoinitiators according to one of claims 1 to 5, having the formula 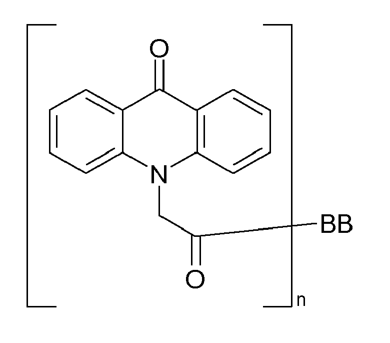 wherein BB is selected from the group consisting of 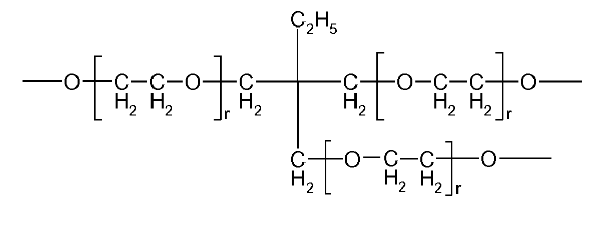 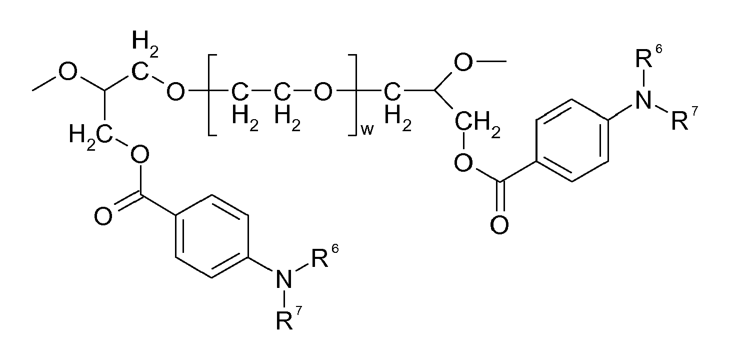 wherein
R6, R7, r and w are as defined above;
n is 2.

13. A process for preparing photoinitiators according to one of claims 1 to 12, comprising the steps of
a) Reacting an optionally substituted thioxanthone or acridone moiety, wherein the thioxanthone moiety comprises at least one hydroxy group, with a compound selected from the group consisting of epichlorohydrine, halo acetic acid esters, halo acetaldehyde, oxirane, and ethylene glycol,
b) Reacting the intermediate obtained in step a) with a respective backbone unit comprising a functional group capable of reacting with said intermediate from step a), or alternately by first reacting the intermediate obtained in step a) with a compound comprising a functional group capable of reacting with said intermediate from step a), and subsequently reacting the thus obtained intermediate with a respective backbone unit;
c) Optionally derivatizing the compound obtained in step b).

14. A coating composition, preferably printing ink, comprising a polymerizable component and at least one photoinitiator according to one of claims 1 to 12.

15. Use of a photoinitiator according to one of claims 1 to 12 for the curing of a coating composition, preferably a printing ink.
